(19) Europäisches Patentamt
European Patent Office
Office européen des brevets

(11) **EP 1 944 059 B1**

## (12) EUROPÄISCHE PATENTSCHRIFT

(45) Veröffentlichungstag und Bekanntmachung des Hinweises auf die Patenterteilung:
**28.08.2013 Patentblatt 2013/35**

(51) Int Cl.:
***A61N 1/36*** *(2006.01)*

(21) Anmeldenummer: **08005369.7**

(22) Anmeldetag: **08.04.2004**

(54) **Vorrichtung zur Desynchronisation von neuronaler Hirnaktivität**

Device for desynchronising neuronal brain activity

Dispositif de désynchronisation de l'activité cérébrale neuronale

(84) Benannte Vertragsstaaten:
**AT BE BG CH CY CZ DE DK EE ES FI FR GB GR HU IE IT LI LU MC NL PL PT RO SE SI SK TR**

(30) Priorität: **17.04.2003 DE 10318071**

(43) Veröffentlichungstag der Anmeldung:
**16.07.2008 Patentblatt 2008/29**

(62) Dokumentnummer(n) der früheren Anmeldung(en) nach Art. 76 EPÜ:
**04726429.6 / 1 613 394**

(73) Patentinhaber: **Forschungszentrum Jülich GmbH**
**52425 Jülich (DE)**

(72) Erfinder: **Tass, Peter**
**52445 Titz (DE)**

(74) Vertreter: **Manitz, Finsterwald & Partner GbR**
**Martin-Greif-Strasse 1**
**80336 München (DE)**

(56) Entgegenhaltungen:
**EP-A2- 1 145 736       WO-A1-97/42990**
**US-A- 5 299 569       US-A- 5 540 734**
**US-A1- 2001 051 819    US-B1- 6 529 774**

• **TASS P A: "Desynchronizing double-pulse phase resetting and application to deep brain stimulation", BIOLOGICAL CYBERNETICS, SPRINGER VERLAG. HEIDELBERG, DE, Bd. 85, Nr. 5, 1. November 2001 (2001-11-01), Seiten 343-354, XP002245895, ISSN: 0340-1200, DOI: 10.1007/S004220100268**

EP 1 944 059 B1

**Beschreibung**

Die Erfindung betrifft eine Vorrichtung zur Desynchronisation von neuronaler Hirnaktivität nach dem Oberbegriff des Anspruchs 1.

Bei Patienten mit neurologischen oder psychiatrischen Erkrankungen, z. B. Morbus Parkinson, essentiellem Tremor, Dystonie oder Zwangserkrankungen, sind Nervenzellverbände in umschriebenen Bereichen des Gehirns, z. B. des Thalamus und der Basalganglien krankhaft aktiv, z. B. übersteigert synchron. In diesem Fall bildet eine große Anzahl von Neuronen synchron Aktionspotentiale aus; die beteiligten Neuronen feuern übermäßig synchron. Beim Gesunden hingegen feuern die Neuronen in diesen Hirngebieten qualitativ anders, z. B. auf unkontrollierte Weise.

Beim Morbus Parkinson verändert die pathologisch synchrone Aktivität z. B. des Thalamus und der Basalganglien die neuronale Aktivität in anderen Hirngebieten, z. B. in Arealen der Großhirnrinde wie dem primär motorischen Cortex. Dabei zwingt die pathologisch synchrone Aktivität im Bereich der Thalamus der Basalganglien beispielsweise den Großhirnarealen ihren Rhythmus auf, so dass schließlich die von diesen Arealen gesteuerten Muskeln phatologische Aktivität, z. B. ein rhythmisches Zittern (Tremor) entfalten.

Bei Patienten, welche medikamentös nicht mehr behandelt werden können, wird je nach Krankheitsbild und je nach dem, ob die Erkrankung einseitig oder beidseitig auftritt, eine Tiefenelektrode einseitig oder beidseitig implantiert. Unter der Haut führt dabei ein Kabel vom Kopf zum sogenannten Generator, welcher ein Steuergerät mit einer Batterie umfasst, und beispielsweise im Bereich des Schlüsselbeins unter der Haut implantiert ist. Über die Tiefenelektroden wird eine Dauerreizung mit einer hochfrequenten periodischen Abfolge (pulse train mit einer Frequenz von > 100 Hz) von Einzelpulsen, z. B. Rechteckpulsen, durchgeführt. Ziel dieser Methode ist es, das Feuern der Neuronen in den Zielgebieten zu unterdrücken. Der Wirkmechanismus, welcher der Standard-Tiefenstimulation zugrunde liegt, ist noch nicht hinreichend geklärt. Die Ergebnisse mehrerer Studien sprechen dafür, dass die Standard-Tiefenstimulation wie eine reversible Läsionierung, d. h. wie eine reversible Ausschaltung des Gewebes, wirkt: Die Standard-Tiefenstimulation unterdrückt das Feuern der Neuronen in den Zielgebieten und/oder in damit verbundenen Hirnarealen.

Nachteilig bei dieser Stimulationsform ist, dass der Energieverbrauch des Generators sehr hoch ist, so dass der Generator inklusive Batterie häufig schon nach ca. ein bis drei Jahren operativ ausgetauscht werden muss. Noch nachteiliger ist, dass die Hochfrequenz-Dauerstimulation als unphysiologischer (unnatürlicher) Input im Bereich des Gehirns, z. B. des Thalamus bzw. der Basalganglien, im Laufe von wenigen Jahren zur Adaptation der betroffenen Nervenzellverbände führen kann. Um den selben Stimulationserfolg zu erzielen, muss dann infolge dieser Adaptation mit höherer Reizamplitude stimuliert werden. Je größer die Reizamplitude ist, desto größer ist die Wahrscheinlichkeit, dass es infolge der Reizung von Nachbararealen zu Nebenwirkungen - wie Dysarthrie (Sprechstörungen), Dysästhesie (zum Teil sehr schmerzhafte Missempfindungen), zerebelläre Ataxie (Unfähigkeit, ohne fremde Hilfe sicher zu stehen) oder Schizophrenie artigen Symptomen etc. - kommt. Diese Nebenwirkungen können vom Patienten nicht toleriert werden. Die Behandlung verliert daher in diesen Fällen nach wenigen Jahren ihre Wirksamkeit.

Deswegen wurde eine andere Methode vorgeschlagen, wie sie in der DE 102 11 766.7 "Vorrichtung zur Behandlung von Patienten mittels Hirnstimulation, ein elektronisches Bauteil sowie die Verwendung der Vorrichtung und des elektronischen Bauteils in der Medizin" beschrieben ist, bei der bedarfsgesteuert Reize im jeweiligen Zielgebiet appliziert werden, welche krankhaft synchronisierte neuronale Aktivität desynchronisieren. Das Ziel dieses Verfahrens/dieser Vorrichtung ist es, das krankhaft synchrone Feuern nicht einfach zu unterdrükken, wie bei der Standard-Tiefenstimulation, sondern, näher an das physiologische, unkorrelierte Feuermuster heran zu bringen. Hierdurch soll einerseits der Stromverbrauch vermindert werden und andererseits Adaptationsprozessen des Nervengewebes, welche über eine Erhöhung der Stimulationsamplitude zu Nebenwirkungen führen können, vorgebeugt werden. Diese bedarfsgesteuerten, desynchronisierenden Verfahren haben aber auch relevante Nachteile.

Nachteile der bedarfsgesteuerten, desynchronisierenden Stimulationsverfahren gemäß DE 102 11 766.7 resultieren aus folgender Tatsache: Um einen synchronisierten Nervenzellverband mit einem elektrischen Reiz zu desynchronisieren, muss ein elektrischer Reiz von bestimmter Dauer präzise zu einer bestimmten Phase der krankhaften rhythmischen Aktivität im Zielareal verabreicht werden. Da solch eine Präzision experimentell zur Zeit noch nicht verlässlich erzielt werden kann, werden zusammengesetzte Stimuli verwendet. Der erste Reiz eines solchen zusammengesetzten Stimulus kontrolliert die Dynamik der zu desynchronisierenden Population durch einen Reset, d. h. einen Neustart, während der zweite Reiz des zusammengesetzten Stimulus den Nervenzellverband in einem vulnerablen Zustand trifft und desynchronisiert. Hierzu ist es aber unabdingbar, dass die Güte der Kontrolle, d. h. die Güte des Resets hinreichend ist, was u. U. bewirken kann, dass ein starker Reiz für den Reset verwendet werden muss. Dies sollte im Sinne einer Verminderung von Nebenwirkungen aber vermieden werden. Noch entscheidender ist aber, dass die gewünschte desynchronisierende Wirkung nur eintritt, wenn die Stimulationsparameter, also die Dauer der einzelnen Reize und insbesondere die Pause zwischen erstem und zweitem Reiz, optimal gewählt werden. Dies hat schwerwiegende Konsequenzen:

   1. Eine zeitaufwendige Kalibrierungs-Prozedur ist erforderlich, die typischerweise länger als 30 Minuten dauert.

2. Infolge der zeitaufwendigen Kalibrierungs-Prozedur kann der Effekt der desynchronisierenden Stimulation gemäß DE 102 11 766.7 nicht zur intraoperativen Auswahl des geeignetsten Zielpunkts für die Tiefenelektrode herangezogen werden. Hierzu müsste für verschiedene Zielpunkte der Effekt der desynchronisierenden Stimulation gemäß DE 102 11 766.7 separat getestet werden, was für jeden Zielpunkt eine separate Kalibrierung erfordern würde; dies würde die Dauer einer Elektrodenimplantation in einer dem Patienten unzumutbaren Weise verlängern.

3. Bei größeren Schwankungen der Netzwerkeigenschaften, d.h. Schwankungen der Parameter, welche die Aktivität der Nervenzellpopulation beschreiben, wie z. B. synaptische Stärken und Feuerraten, muss neu kalibriert werden, was bedeutet, dass während der Kalibrierung kein therapeutischer Effekt erzielt werden kann.

4. Da die desynchronisierende Stimulation gemäß DE 102 11 766.7 nur dann wirkt, wenn die Frequenz der zu desynchronisierenden Neuronenpopulation keinen größeren Schwankungen unterliegt, ist sie nicht anwendbar bei Erkrankungen mit kurzzeitig auftretenden Epochen krankhaft übersteigerter synchroner Aktivität mit stark variierender Frequenz, also zum Beispiel bei Epilepsien.

Aus der Schrift "Desynchronizing double-pulse phase resetting and application to deep brain stimulation" von P. Tass, Biol. Cybern. 85, 2001, Seiten 343 bis 354 ist ein Verfahren zur Phasenrücksetzung einer Neuronenpopulation bekannt. Die Schriften EP 1 145 736 A2, US 5 299 569 A, US 5 540 734 A, US 2001/051819 A1, US 6 529 774 B1 und WO 97/42990 A1 offenbaren weitere Anwendungen von Neurostimulatoren.

Es ist daher die Aufgabe der Erfindung eine Vorrichtung zur Desynchronisation von neuronaler Hirnaktivität zu schaffen, mit der bei der Behandlung von Patienten mit einer Elektrodenstimulation eine Adaptation an einen unphysiologischen Dauerreiz unterbunden wird. Es sollen langwierige Kalibrierungsvorgänge verhindert werden und die Stimulation soll auch dann erfolgen können, wenn die Hauptfrequenz-Komponente der pathologisch rhythmischen Aktivität starken Schwankungen unterliegt. Die erfindungsgemäße Stimulationsvorrichtung soll stromsparend funktionieren, so dass die in den Patienten implantierten Batterien seltener operativ ausgetauscht werden müssen.

Ausgehend vom Oberbegriff des Anspruchs 1 wird die Aufgabe erfindungsgemäß gelöst durch die im kennzeichnenden Teil des Anspruchs 1 angegebenen Merkmale. Überraschenderweise wird die Aufgabe dadurch gelöst, dass mindestens zwei Teilbereiche eines Hirnareals oder mindestens zwei funktionell zusammengehörige Hirnareale mit N Elektroden in ihrer Aktivität jeweils resettet, das heißt bezüglich ihrer Phase rückgesetzt werden, wonach sich bei einer erkrankten Person überraschenderweise eine Desynchronisation in der betroffenen Neuronenpopulation einstellt und die Symptomatik unterdrückt wird. Erfindungsgemäß sind aufeinander folgende und von unterschiedlichen der N Elektroden verabreichte phasenrücksetzende Reize um T/N zeitverschoben, wobei T die Periode der rhythmischen, zu desynchronisierenden Aktivität ist.

Mit der erfindungsgemäßen Vorrichtung ist es nunmehr möglich, Patienten mittels einer Mehrelektroden-Stimulation zu behandeln, (i) ohne dass dabei eine Adaptation an einen unphysiologischen Dauerreiz stattfindet, (ii) ohne dass langwierige Kalibrierungsvorgänge notwendig wären, (iii) auch wenn die Hauptfrequenz-Komponente der pathologisch rhythmischen Aktivität starken Schwankungen unterliegt. Hierdurch können die oben genannten Nebenwirkungen vermindert oder unterbunden werden. Die erfindungsgemäße Vorrichtung ermöglicht es, den Effekt der mit der erzielten desynchronisierenden Stimulation intraoperativ zur Auswahl des am besten geeigneten Zielpunkts für die Tiefenelektrode zu nutzen. Hierzu wird während der Implantation der Tiefenelektrode im Bereich des anatomisch vorberechneten Zielpunkts in mm-Schritten vorangehend eine Testreizung mit der erfindungsgemäßen Vorrichtung durchgeführt. Der Zielpunkt, bei welchem sich der beste therapeutische Effekt erzielen lässt, wird als Zielpunkt für die dauerhafte Implantation gewählt. Außerdem können neben den obengenannten Erkrankungen die häufig anhaltende pathologisch synchrone Aktivität mit relativ konstanter Frequenz aufweisen, auch Erkrankungen behandelt werden, bei denen es nur intermittent (kurzzeitig auftretend) zu pathologisch synchroner Aktivität kommt. Eine Hauptindikation ist dabei die Behandlung von medikamentös nicht (mehr) behandelbaren Epileptikern. Die erfindungsgemäße Vorrichtung kann beispielsweise bei den Krankheiten Morbus Parkinson, essentieller Tremor, Dystonie, Epilepsie und Zwangserkrankungen eine Desynchronisation bewirken.

Die erfindungsgemäße Vorrichtung arbeitet Strom sparend, so dass im Patienten implantierte Batterien seltener ausgetauscht werden müssen.

Vorteilhafte Weiterbildungen der Erfindung sind in den Unteransprüchen angegeben.

Die Figuren zeigen beispielhafte Ausführungsformen der Erfindung:

Es zeigt:

Fig.1:     Eine erfindungsgemäße Vorrichtung

Fig.2a:     Zeitgang der Amplitude des über Sensor 3 gemessenen lokalen Feldpotentials während des bedarfsgesteuerten Timings.

Fig.2b:     Zeitgang des über Sensor 3 gemessenen Entladungsmusters der Nervenzellen während des bedarfsgesteuerten Timings.

Fig.3a:    Zeitgang der Amplitude des über Sensor 3 gemessenen lokalen Feldpotentials während wiederkehrender Applikation mit bedarfsgesteuerter Reizstärke.

Fig.3b:    Zeitgang des über Sensor 3 gemessenen Entladungsmusters der Nervenzellen während wiederkehrender Applikation mit bedarfsgesteuerter Reizstärke.

Fig.4a-d:    Beispiel für eine Reizapplikation mit 4 Elektroden.

Fig.5a-d:    Beispiel für eine zeitversetzte Applikation von identischen Hochfrequenzpulszügen über 4 Elektroden.

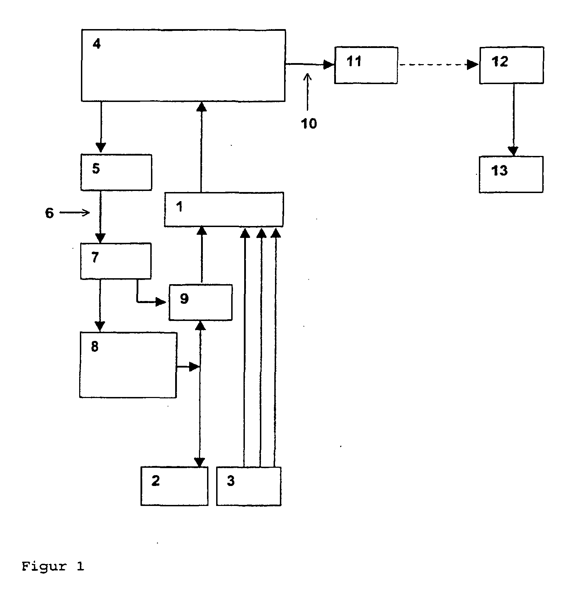

Die Vorrichtung gemäß Figur 1 umfasst einen Trennverstärker 1, an den mindestens zwei Elektroden 2 sowie Sensoren 3 zur Erfassung von physiologischen Messsignalen angeschlossen sind. Der Trennverstärker steht weiterhin mit einer Einheit 4 zur Signalverarbeitung und Steuerung in Verbindung, welche an einen optischen Sender für die Stimulation 5 angeschlossen ist. Der optische Sender 5 ist über Lichtwellenleiter 6 mit einem optischen Empfänger 7 verbunden, welcher mit einer Stimulatoreinheit 8 zur Signalerzeugung in Verbindung steht. Die Stimulatoreinheit 8 für die Signalerzeugung steht mit mindestens zwei Elektroden 2 in Verbindung. Am Eingangsbereich der Elektroden 2 in den Trennverstärker 1 befindet sich ein Relais 9 oder Transistor. Die Einheit 4 steht über eine Leitung 10 mit einem Telemetriesender 11 in Verbindung, welcher mit einem Telemetrieempfänger 12 in Verbindung steht, der sich außerhalb des zu implantierenden Geräts befindet und an den ein Mittel zur Visualisierung, Verabreitung und Speicherung der Daten 13 angeschlossen ist. Als Sensoren 3 können beispielsweise epikortikale Elektroden, Tiefenelektroden, Hirnelektroden oder periphere Elektroden eingesetzt werden.

Bei den Elektroden 2 handelt es sich um jeweils mindestens zwei Drähte, an deren Enden eine Potentialdifferenz zum Zwecke der Stimulation angelegt wird. Es kann sich dabei um Makro- oder Mikroelektroden handeln. Alternativ kann es sich bei den Elektroden 2 auch um jeweils einzelne Drähte handeln. In diesem Fall wird zum Zwecke der Stimulation jeweils eine Potentialdifferenz zwischen einem einzelnen Draht und dem metallischen Teil des Gehäuses des Generators angelegt. Zusätzlich, aber nicht zwingend, kann über die Elektroden 2 eine Potentialdifferenz gemessen werden, um eine pathologische Aktivität festzustellen. In einer weiteren Ausführungsform können die Elektroden 2 auch aus mehr als zwei einzelnen Drähten bestehen, die sowohl für die Ermittlung eines Messsignals im Gehirn, als auch für die Stimulation herangezogen werden können. Beispielsweise können vier Drähte in einem Leiterkabel untergebracht sein, wobei zwischen verschiedenen Enden eine Potentialdifferenz angelegt oder gemessen werden kann. Hierdurch lässt sich die Größe des abgeleiteten bzw. stimulierten Zielgebietes variieren. Die Anzahl der Drähte, aus welchen sich die Elektrode aufbaut, ist nach oberen Werten hin lediglich durch die damit verbundene Dicke des in das Gehirn einzuführenden Kabels begrenzt, so dass möglichst wenig Hirnmaterial beschädigt werden soll. Handelsübliche Elektroden umfassen vier Drähte, es können jedoch auch fünf, sechs oder mehr Drähte, aber auch nur drei Drähte umfasst sein.

Für den Fall, dass die Elektroden 2 mehr als zwei Drähte umfassen, können mindestens zwei dieser Drähte auch als Sensor 3 fungieren, so dass in diesem Spezialfall eine Ausführungsform vorliegt, bei der die Elektroden 2 und der Sensor 3 in einem einzigen Bauteil vereint sind. Die Drähte der Elektroden 2 können unterschiedliche Längen haben, so dass sie in verschiedene Hirntiefen eindringen können. Bestehen die Elektroden 2 aus n Drähten, so kann eine Stimulation über mindestens ein Paar von Drähten erfolgen, wobei bei der Paarbildung jede Unterkombination von Drähten möglich ist. Neben diesem Bauteil können zusätzlich nicht mit den Elektroden 2 baulich vereinte Sensoren 3 vorhanden sein.

Erfindungsgemäß ist die Vorrichtung mit Mitteln ausgestattet, welche die Signale der Elektroden 2 und oder der Sensoren 3 als pathologisch erkennen und im Falle des Vorliegens eines pathologischen Musters über die Elektroden 2 Reize abgeben, die bewirken dass die pathologische neuronale Aktivität in den von den einzelnen Elektroden 2 stimulierten Subpopulationen einen Reset erfährt, so dass die neuronale Aktivität der Gesamtpopulation desynchronisiert wird und somit der natürlichen, physiologischen Aktivität näher kommt. Die pathologische Aktivität unterscheidet sich von der gesunden Aktivität durch eine charakteristische Veränderung ihres Musters und/oder ihrer Amplitude und/oder ihres Frequenzgehalts.

Die Mittel zum Erkennen des pathologischen Musters sind dabei ein Rechner, der die gemessenen Signale der Elektroden 2 und/oder des Sensors 3 verarbeitet und mit im Rechner gespeicherten Daten vergleicht. Der Rechner verfügt über einen Datenträger, welcher Daten speichert. Diese können im Rahmen der Kalibrierung und/oder Steuerung gemäß Abschnitt 5 ermittelt werden.

Die erfindungsgemäße Vorrichtung umfasst daher in einer möglichen Ausführungsform als Einheit 4 zur Signalverarbeitung und/oder Steuerung/Regelung einen Rechner, welcher einen Datenträger beinhaltet, der die Daten des Krankheitsbildes trägt und mit den Messdaten vergleicht. Unter den Daten des Krankheitsbildes werden für die Stimulation relevante Parameter und Messgrößen verstanden, zum Beispiel die Momentanfrequenz des über Sensor 3 gemessenen Feedback-Signals, der für die Verfahrensweise des bedarfsgesteuerten Timings notwendigen Schwellenwerts, die Stimulationsparameter, welche die Reizstärke festlegen, zum Beispiel die Amplitude und Anzahl der Einzelpulse eines Hochfrequenz-Pulszuges. Insgesamt sollen alle für die jeweilige Verfahrensweise der erfindungsgemäßen Vorrichtung relevanten Parameter für die Art und Stärke der Stimuli, als auch deren zeitliche Abstände sowie Information zur elektrodenspezifischen Applikation als auch die für die bedarfsgesteuerte Funktionsweisen relevanten, über Sensor 3 ermittelten Messwerte bzw. daraus abgeleiteten Parameter abgespeichert werden. In Abhängigkeit vom Auftreten und der Ausprägung pathologischer Merkmale im Feedback-Signal wird in der Ausführungsform des in Abschnitt 4.3. beschriebenen bedarfsgesteuerten Timings ein Reizsignal an die Elektroden 2 abgegeben, so dass eine Stimulation des Hirngewebes erfolgt. Die erfindungsgemäße Vorrichtung verfügt über Mittel zum Erkennen des Auftretens und/oder der Ausprägung der pathologischen Merkmale in dem über Sensor 3 gemessenen Feedback-Signal. Die Steuereinheit 4 ist so programmiert, dass in der Ausführungsform des in Abschnitt 4.3. beschriebenen Timings zu einem von Steuereinheit 4 ein Reizsignal generiert und an die Elektroden 2 abgegeben wird. Die Steuereinheit 4 ist so programmiert, dass in der Ausführungsform der in Abschnitt 4.4. beschriebenen periodischen Stimulation mit bedarfsgesteuerter Reizstärke zu von Steuereinheit 4 bestimmten, vorzugsweise periodisch aufeinanderfolgenden Zeitpunkten ein Reizsignal mit einer von Steuereinheit 4 berechneten Stärke generiert und an die Elektroden 2 abgegeben wird. In einer weniger bevorzugten Ausführungsform arbeitet die Steuerung ohne Bedarfssteuerung, das heißt, ohne Feedback-Kontrolle und generiert, wie in Abschnitt 4.2. beschrieben, Reizsignale, welche an die Elektroden 2 abgegeben werden.

Die Steuereinheit 4 kann beispielsweise einen Chip oder eine andere elektronische Vorrichtung mit vergleichbarer Rechenleistung umfassen.

Die Steuereinheit 4 steuert die Elektroden 2 vorzugsweise in folgender Weise an. Die Steuerdaten werden von der Steuereinheit 4 an einen optischen Sender für die Stimulation 5 weitergegeben, welcher über den Lichtleiter 6 den optischen Empfänger 7 ansteuert. Durch das optische Einkoppeln von Steuersignalen in den optischen Empfänger 7, wird eine galvanische Entkopplung der Stimulationssteuerung von den Elektroden 2 bewirkt. Dies bedeutet, dass eine Einstreuung von Störsignalen von der Einheit zur Signalverarbeitung und Steuerung 4 in die Elektroden 2 verhindert wird. Als optischer Empfänger 7 kommt beispielsweise eine Photozelle in Betracht. Der optische Empfänger 7 gibt die über den optischen Sender für die Stimulation 5 eingegebenen Signale an die Stimulatoreinheit 8 weiter. Über die Stimulatoreinheit 8 werden dann gezielte Stimuli über die Elektroden 2 an die Zielregion im Gehirn weitergegeben. Für den Fall, dass über die Elektroden 2 auch gemessen wird, wird ausgehend vom optischen Sender für die Stimulation 5 über den optischen Empfänger 7 auch ein Relais 9 angesteuert, wodurch die Einstreuung von Störsignalen verhindert wird. Das Relais 9 oder der Transistor stellt sicher, dass die neuronale Aktivität unmittelbar nach jedem Stimulus wieder gemessen werden kann, ohne dass der Trennverstärker übersteuert. Die galvanische Entkopplung muss nicht zwingend durch eine optische Einkopplung der Steuersignale erfolgen, vielmehr können auch andere alternative Steuerungen verwendet werden. Diese können beispielsweise akustische Einkopplungen zum Beispiel im Ultraschallbereich sein. Eine störungsfreie Steuerung kann auch beispielsweise unter Zuhilfenahme geeigneter analoger oder digitaler Filter realisiert werden.

Weiterhin steht die erfindungsgemäße Vorrichtung vorzugsweise mit Mitteln zur Visualisierung und Verarbeitung der Signale sowie zur Datensicherung 13 über den Telemetrieempfänger 12 in Verbindung. Dabei kann die Einheit 13 über die unten erwähnten Verfahren zur Datenanalyse verfügen.

Weiterhin kann die erfindungsgemäße Vorrichtung über den Telemetrieempfänger 13 mit einer zusätzlichen Referenzdatenbank in Verbindung stehen, um beispielsweise den ordnungsgemäßen Betrieb des Gerätes zu überwachen und ggf. die in Abschnitt 5.1.2. beschriebenen Steuermechanismus durch Modifikation der Parameter effizienter auszugestalten. Zum Beispiel kann, wie in Abschnitt 5.1.2.2.2.1. beschrieben, die Mindestanzahl der Einzelpulse eines Hochfrequenz-Pulszugs erhöht bzw. erniedrigt werden, um die Stärke der desynchronisierenden Wirkung der Stimulation zu erhöhen bzw. zu erniedrigen.

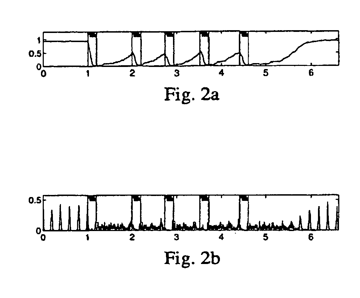

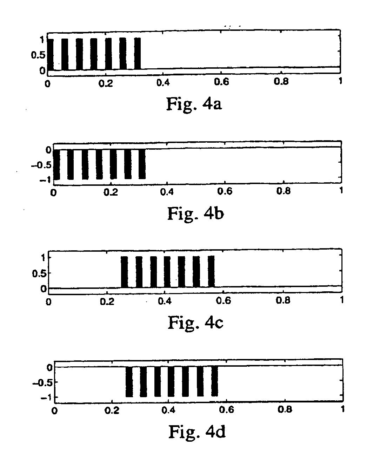

In den Figuren 2a, b bezeichnen die Abszissen die Zeitachsen in Sekunden, während auf den Ordinaten die Amplitude des lokalen Feldpotentials (Fig.2a) bzw. das neuronale Entladungsmuster (Fig.2b) jeweils in willkürlichen Einheiten aufgetragen sind. Die Amplitude des über Sensor 3 gemessenen lokalen Feldpotentials (Fig. 2a) dient als Feedback-Signal für das bedarfsgesteuerte Timing. Immer, wenn eine Schwelle des Feedback-Signals erreicht wird, erfolgt die nächste Stimulation mit demselben Reiz. Die vertikalen Striche symbolisieren Beginn und Ende des über vier Elektroden 2 applizierten Reizes. Letzter ist in Figur 4a-d dargestellt und besteht aus zwei zeitversetzten Paaren von Hochfrequenzpulszügen. Jedes Paar besteht dabei aus zwei Hochfrequenzpulszügen unterschiedlicher Polarität. Die beiden zwischen den vertikalen Strichen gelegenen Balken in den Figuren 2a, b symbolisieren die beiden Paare von Hochfrequenzpulszüge: Der obere Balken entspricht dem in den Figuren 4a, b dargestellten Paar, der untere Balken gehört zu dem in den Figuren 4c, d aufgeführten Paar.

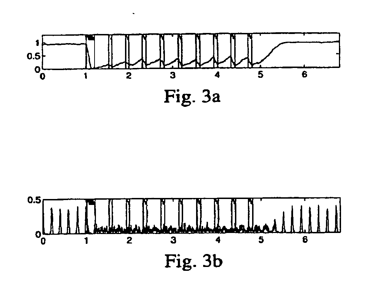

In den Figuren 3a, b bezeichnen die Abszissen die Zeitachsen in Sekunden, während auf den Ordinaten die Amplitude des lokalen Feldpotentials (Fig.3a) bzw. das neuronale Entladungsmuster (Fig.3b) in willkürlichen Einheiten aufgetragen sind. Die Amplitude des über Sensor 3 gemessenen lokalen Feldpotentials (Fig.3a) dient als Feedback-Signal für die periodische Applikation mit bedarfsgesteuerter Reizstärke. Die in den Figuren 4a-d dargestellten Hochfrequenzpulszüge werden periodisch appliziert, wobei innerhalb eines über die vier Elektroden 2 verabreichten Gesamtreizes die Länge aller vier Hochfrequenzpulszüge identisch ist und an das vor Reizapplikation gemessene lokale Feldpotential angepasst werden. Die vertikalen Striche symbolisieren Beginn und Ende des über vier Elektroden 2 applizierten Reizes. Letzterer ist in Figur 4a-d dargestellt und besteht aus zwei zeitversetzten Paaren von Hochfrequenzpulszügen. Jedes Paar besteht dabei aus zwei Hochfrequenzpulszügen unterschiedlicher Polarität. Die beiden zwischen den vertikalen Strichen gelegenen Balken in den Figuren 3a, b symbolisieren die beiden Paare von Hochfrequenzpulszügen: Der obere Balken entspricht dem in den Figuren 4a, b dargestellten Paar, der untere Balken gehört zu dem in den Figuren 4c, d aufgeführten Paar. Die bedarfsgesteuert gewählte Länge der Hochfrequenzpulszüge wird über die Länge des oberen und unteren Balkens in den Figuren 3a, b symbolisiert.

In den Figuren 4a-d ist die Abszisse die Zeitachse in Sekunden, während auf der Ordinate die Stärke der Einzelpulse, zum Beispiel im Sinne des applizierten Stroms, in willkürlichen Einheiten dargestellt ist. Zur besseren Visualisierung sind die Einzelpulse schwarz ausgefüllt. Über die ersten beiden Elektroden 2 wird derselbe Hochfrequenzpulszug, aber mit unterschiedlicher Polarität appliziert (Fig.4a,b). Das selbe Paar von Hochfrequenzpulszügen wird zeitlich verzögert über die dritte und vierte Elektrode 2 appliziert (Fig.4c, d).

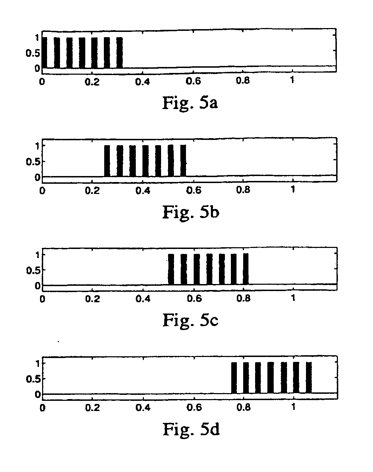

In den Figuren 5a-d ist die Abszisse die Zeitachse in Sekunden, während auf der Ordinate die Stärke der Einzelpulse, zum Beispiel im Sinne des applizierten Stroms, in willkürlichen Einheiten dargestellt ist. Zur besseren Visualisierung sind die Einzelpulse schwarz ausgefüllt. Über die ersten beiden Elektroden 2 wird derselbe Hochfrequenz-Pulszug mit derselben Polarität appliziert (Fig.5a, b). Das selbe Paar von Hochfrequenzpulszügen wird zeitlich verzögert über die dritte und vierte Elektrode 2 appliziert (Fig.5c, d).

Im Folgenden soll die erfindungsgemäße Vorrichtung und deren Funktionieren beispielhaft erläutert werden.

Die erfindungsgemäße Vorrichtung und die Steuerung sind mit Mitteln ausgestattet, die alle Schritte des erfindungsgemäßen Behandlungsverfahrens durchführen können. Mit den offenbarten Verfahrensschritten sollen daher implizit auch Mittel zur Durchführung des Verfahrensschrittes offenbart sein. Die Verfahrensschritte stellen somit auch gleichzeitig die funktionalisierten Vorrichtungsmerkmale dar.

Erfindungsgemäß werden die Elektroden in die Hirnregion eingebracht, welche für die Ausbildung des Krankheitsbildes verantwortlich ist. Entweder direkt in das Gebiet oder in eine oder mehrere mit diesem Gebiet verbundenen Nervenzellpopulationen oder Nervenfaserbündel werden erfindungsgemäß mindestens zwei bevorzugt vier aber auch drei oder mehr Elektroden eingebracht. Die Anzahl der Elektroden ist lediglich dadurch begrenzt, dass keine beliebig hohe Dichte an Elektroden in einer Hirnregion vorhanden sein soll, damit das Gewebe nicht unnötig beschädigt wird und vor allem das Blutungsrisiko beim Einführen der Elektroden vermindert wird. Jedenfalls soll die Anzahl der in die Region eingebrachten Elektroden $N$ sein, mit $N \geq 2$. Jede Elektrode gibt dabei in ihrer Umgebung ein Signal ab, welches entweder direkt in ihrem Umfeld oder über ein Nervenfaserbündel fortgeleitet in einem anderen Areal einen Reset der neuronalen Aktivität bewirkt. Die zu einem Reset befähigten Elektrodensignale sind dem Fachmann bekannt, es können beispielhaft Einzelpulse oder Hochfrequenzpulszüge mit einer Pulsrate von mehr als 100 Hz genannt werden. Die erfindungsgemäße Vorrichtung verfügt daher über eine Steuerung, welche mindestens zwei Elektroden 2 so ansteuert, dass sie in ihrem näheren Umfeld und/oder durch Weiterleitung der Stimulation über ein Faserbündel in einem anderen Gehirnareal einen Reset bewirken.

Erfindungsgemäß werden N Elektroden mit $N \geq 2$ vorzugsweise so angesteuert, dass eine zeitliche Phasenverschiebung der einzelnen Elektrodensignale um T/N vorliegt, sofern die stimulierenden Elektroden 2 sich in dem zu desynchronisierenden Areal befinden. T ist hierbei, wie unten beschrieben, die Periode der rhythmischen, zu desynchronisiernden Aktvität. Falls sich mindestens eine der stimulierenden Elektroden 2 nicht in dem zu desynchronisiernden Areal befindet, ist bei der Ansteuerung einer solchen Elektrode 2 die Laufzeit zwischen dem Reizort, und dem Ort der hierdurch beeinflussten Neuronenpopulation zu berücksichtigen. Dies wird in Abschnitt 5.2 beschrieben. Demgemäß verfügt die erfindungsgemäße Vorrichtung über eine Steuerung, die bei N Elektroden vorzugsweise ein um im wesentlichen ein N-tel der Periode der zu desynchronisierenden Aktivität zeitverschobenes Resetsignal erzeugt. Die zeitliche Phasenverschiebung ist dabei vorzugsweise im wesentlichen äquidistant. Unter Phasenverschiebung wird hier die Differenz zwischen den Phasen der durch die unterschiedlichen Elektroden 2 beeinflussten, zu desynchronisierenden rhythmischen Aktivität verstanden. Überraschenderweise wird bei diesem äquidistant phasenverschobenen Reset der von den N Elektroden 2 jeweils beeinflussten Neuronenpopulation insgesamt eine Desynchronisation der gesamten, zu desynchronisierenden Neuronenpopulation bewirkt, was mit einer Unterdrückung der phatologischen Symptome einhergeht. Befindet sich mindestens eine Elektrode 2 außerhalb des zu desynchronisierenden Areals, so müssen Effekte der indirekten Stimulation, wie sie in Abschnitt 3.2 beschrieben sind berücksichtigt werden. Dies wird im Detail in den Abschnitten 3.3., 3.4 und 5 erläutert.

Mit dem neuen Verfahren/der neuen Vorrichtung wird die Desynchronisation im Vergleich zum oben genannten Stand der Technik qualitativ anders erzielt. Anstatt den krankhaft synchronen Nervenzellverband gezielt in einer vulnerablen Phase seines Rhythmus zu treffen, wird der betroffene Nervenzellverband einfach an mehreren Orten zeitlich koordiniert in einer Weise stimuliert, dass eine Desynchronisation auftritt. Hierbei können an den einzelnen Reizorten entweder elektrische Einzelpulse, Niederfrequenz-Reizfolgen oder Hochfrequenz-Reizfolgen verwendet werden. Es muss an mindestens zwei, vorzugsweise mehr als zwei Reizorten stimuliert werden. Wird an N Reizorten stimuliert, wird die gesamte zu desynchronisierende Nervenzellpopulation in N (im Phasenzyklus) im wesentlichen äquidistante Subpopulationen aufgeteilt. Das heißt, dass die Phasen der neuronalen Aktivität der Subpopulationen in im wesentlichen äquidistanten Schritten von $2\pi/N$ aufeinander folgen. $2\pi$ ist dabei die Länge einer Periode, die oben auch als Phasenzyklus bezeichnet wird. Dabei wird ausgenutzt, dass es durch die krankhaft gesteigerte Interaktion zwischen den Neuronen dann zu einer Desynchronisation kommt. Man nutzt hierbei einen überraschenderweise vorhandenen Selbstorganisations-Prozess aus, welcher für die krankhafte Synchronisation verantwortlich ist. Derselbe bewirkt, dass auf eine äquidistante Aufteilung in Subpopulationen, das heißt eine Aufteilung einer Gesamtpopulation in Subpopulationen, deren Phasen äquidistant verteilt sind, eine Desynchronisation folgt. Im Gegensatz dazu würde ohne krankhaft gesteigerte Interaktionen keine Desynchronisation erfolgen. Man nutzt anschaulich gesprochen die Energie des zu beeinflussenden Systems aus, um mit minimalem Eingriff einen therapeutischen Effekt zu erzielen. Eine äquidistante Aufteilung in Subpopulationen ist viel einfacher zu erzielen als eine komplette Desynchronisation mit den im Stand der Technik beschriebenen Methoden. Die besten Ergebnisse werden erzielt, wenn eine äquidistante Phasenverschiebung oder eine im wesentlichen äquidistante Phasenverschiebung der phasenrücksetzenden Reize appliziert werden. Es werden aber auch noch Behandlungserfolge erzielt, wenn die über die Elektroden 2 abgegebenen Reize die Phasen der stimulierten Subpopulation zumindest teilweise gegeneinander verschieben. Die Behandlungsergebnisse werden aber um so besser, je mehr die erzielte Phasenverschiebung einer äquidistanten Phasenverschiebung nahe kommt.

## 1. Mechanismus der Stimulation:

Ziel der Stimulation ist es, einer krankheitsbedingt vorhandenen Synchronisation in einer Nervenzellpopulation durch Desynchronisation entgegenzuwirken. Hierzu wird die zu desynchronisierende Nervenzellpopulation an mindestens zwei Stellen durch die erfindungsgemäße phasenverschobene Reizung an unterschiedlichen Orten des Gehirns so beeinflusst, dass sich vorübergehend mindestens zwei Subpopulationen der gesamten Nervenzellpopulation bilden. Bedingt durch die krankhafte Interaktion zwischen den Nervenzellen ist der durch die Stimulation erzeugte Zustand mit mindestens zwei Subpopulationen instabil, und die gesamte Nervenzellpopulation nähert sich sehr schnell einem Zustand kompletter Desynchronisation. Der gewünschte Zustand, das heißt die komplette Desynchronisation, ist somit nach der Applikation eines Reizes nicht sofort vorhanden, sondern stellt sich krankheitsbedingt typischerweise, während weniger Perioden, häufig in weniger als einer Periode ein. Mit den nach dem Stand der Technik beschriebenen Desynchronisationsmethoden wird die zu desynchronisierende Nervenzellpopulation unmittelbar in einen desynchronisierten Zustand gebracht. Dies gelingt aber nur bei adäquater Wahl der Stimulationsparameter, wobei diese präzise kalibriert sein müssen und nur geringe Fehlertoleranz aufweisen. Die erfindungsgemäße Vorrichtung hingegen stimuliert die zu desynchronisierende Nervenzellpopulation auf qualitativ unterschiedliche Weise: Durch die koordinierte zeitlich Stimulation von Subpopulationen wird die zu desynchronisierende Nervenzellpopulation in mindestens zwei Subpopulationen aufgespalten. Dieser Prozess funktioniert für einen großen Bereich der modifizierbaren Stimulationsparameter, benötigt keine aufwendige Kalibrierung und verfügt über eine große Fehlertoleranz. Der Grund hierfür ist, dass anders als beim im Einleitungsteil beschriebenen Stand der Technik nicht eine vulnerable Phase, welche ca. 5 % einer Periode des zu desynchronisierenden Rhythmus entspricht, getroffen werden muss. Vielmehr wirkt die Stimulation unabhängig vom dynamischen Anfangszustand.

## 2. Art der Einzelreize:

Als Einzelreiz wird im Folgenden ein Reiz bezeichnet, der über eine einzelne Elektrode 2 appliziert wird. Im Gegensatz dazu wird im Folgenden unter einem Einzelpuls ein einzelner pulsförmiger monophasischer oder biphasischer Reiz verstanden. Ein Einzelpuls kann entweder als Einzelreiz verabreicht werden oder Teil eines Hochfrequenz- oder Niederfrequenz-Pulszugs sein. Für die zeitlich koordinierte Stimulation über mindestens zwei Elektroden werden beispielsweise folgende, dem Fachmann bekannte Einzelreize verwendet:

a) Elektrischer monophasischer oder biphasischer Einzelpuls,
b) elektrische Hochfrequenzpulszüge mit einer Pulsrate von vorzugsweise mehr als 100 Hz, wobei die Einzelreize des Pulszugs monophasische oder biphasische Einzelpulse sein können,
c) elektrische Niederfrequenzpulszüge, wobei mit einer Pulsrate f in der Größenordnung der Frequenz g des zu desynchronisierenden Rhythmus mono- oder biphasische Einzelpulse oder ein kurzer Hochfrequenzpulszug bestehend aus wenigen - vorzugsweise 1 bis zu 20 - mono- oder biphasischen Einzelpulsen appliziert wird. Hierbei ist die Frequenz der Pulsrate des Niederfrequenzpulszugs vorzugsweise im wesentlichen durch das Verhältnis $f/g = n/m$ gegeben, wobei n und m kleine ganze Zahlen, vorzugsweise 1, 2 oder 3, sind.
d) Neben der unter b) und c) beschriebenen im wesentlichen periodischen Abfolge der Einzelpulse in einem Hochfrequenzpulszug beziehungsweise Niederfrequenzpulszug, können die Zeitpunkte der Applikation der Einzelpulse in einem Pulszug auch stochastisch und/oder deterministisch variiert werden.

Unter zeitlich koordinierter Stimulation wird hierbei verstanden, dass die Einzelreize über die jeweilige Elektrode 2 zu jeweils geeigneten, möglicherweise unterschiedlichen Zeiten - wie in Abschnitt 4.1. beschrieben - appliziert werden, um zwischen den stimulierten Subpopulationen der zu desynchronisierenden Neuronenpopulation für die therapeutische Wirkung günstige Phasendifferenzen zu erzeugen. Hierzu verfügt die erfindungsgemäße Vorrichtung über Mittel, welche die beschriebenen elektrischen monophasischen und/oder biphasischen Einzelpulse und/oder elektrische Hochfrequenzpulszüge und/oder elektrische Niederfrequenzpulszüge der beschriebenen Art applizieren. Die Mittel sind Elektroden 2 sowie eine Steuerung 4, welche Steuersignale an die Elektroden 2 für die Abgabe dieser Reize abgibt.

Als Gesamtreiz werden die über die Elektroden 2 applizierten Einzelreize bezeichnet, welche gemäß des Wirkmechanismus der erfindungsgemäßen Vorrichtung in der zu desynchronisierenden Neuronenpopulation eine Desynchronisation hervorrufen. Beispiele für Gesamtreize sind in den Figuren 4 a-d und 5 a-d gezeigt. Im Rahmen eines Gesamtreizes wird vorzugsweise über jede Elektrode ein Einzelreiz abgegeben.

Bei repetitiver Applikation von Gesamtreizen können die im Rahmen eines Gesamtreizes angesteuerten Elektroden 2 variiert werden. Insbesondere kann die Teilmenge der Elektroden 2, die beim jeweiligen Gesamtreiz angesteuert wird, mittels eines stochastischen und/oder deterministischen Algorithmus ausgewählt werden.

3. Anzahl und räumliche Anordnung der Elektroden:

3.1. Anzahl der Elektroden:

Die Anzahl der Elektroden ergibt sich als Kompromiss aus zwei gegenläufigen Bestrebungen:

Einerseits sollte die zu desynchronisierende Neuronenpopulation durch die Stimulation in möglichst viele funktionelle Subpopulationen aufgeteilt werden. Dies geht um so besser, je mehr Elektroden für die Stimulation verwendet werden. Andererseits soll die Anzahl der zu implantierenden Elektroden möglichst gering gehalten werden, um unnötigen Gewebsschädigungen und vor allem einer Hirnblutung während der Implantation vorzubeugen. Es können beispielsweise mindestens 2 Elektroden eingesetzt werden. Es können auch 3 Elektroden verwendet werden. Besonders bevorzugt ist die Verwendung von 4 Elektroden, da die Desynchronisation bei 4 Elektroden ausgeprägter und länger andauernd wirkt. Mit der Zunahme der Anzahl der Elektroden auf beispielsweise 5, 6, 7, 8, 9 bis zu 100 und mehr wird der Desynchronisationseffekt bezüglich Ausprägung und Dauer verbessert. Die Verwendung von einer größeren Anzahl von Elektroden, wie beispielsweise 100 Elektroden, kann nur realisiert werden, wenn Mikroelekroden bzw. moderne Neurochiptechnologien verwendet werden.

3.2. Definition der Begriffe:

Im Folgenden wird unter der Zielpopulation die unmittelbar durch eine implantierte Elektrode stimulierte Nervenzellpopulation verstanden.

Eine Zielpopulation wird durch eine in ihr oder nahe bei ihr implantierte Elektrode direkt stimuliert.

Die Nervenzellpopulation, welche krankhaft synchron aktiv ist, wird als zu desynchronisierendes Areal oder als zu desynchronisierende Nervenzellpopulation oder als zu desynchronisierende Neuronenpopulation bezeichnet. Letztere ist nicht an anatomische Grenzen gebunden. Vielmehr kann darunter auch mindestens eine Komponente, bestehend aus der Gruppe

- mindestens ein Teil von mindestens einem anatomischen Areal,
- mindestens ein vollständiges anatomisches Areal, verstanden werden.

Das zu desynchronisierende Areal kann entweder direkt oder indirekt stimuliert werden.

Direkte Stimulation über eine Stimulationselektrode 2:

In diesem Fall befindet sich die Stimulationselektrode 2 in dem zu desynchronisierenden Areal. Diese Elektrode 2 beeinflusst dabei die Zielpopulation, welche sich in dem zu desynchronisierenden Areal befindet.

Indirekte Stimulation über eine Stimulationselektrode 2:

In diesem Fall wird das zu desynchronisierende Areal mittels Elektrode 2 nicht direkt stimuliert. Vielmehr wird über die Stimulationselektrode 2 eine Zielpopulation oder ein Faserbündel, welche mit dem zu desynchronisierenden Areal funktionell eng verbunden sind, stimuliert. Hierbei wird der Stimulationseffekt auf das zu desynchronisierende Areal vorzugsweise über anatomische Verbindungen fortgeleitet. Für die indirekte Stimulation soll als Oberbegriff für Zielpopulation und Faserbündel der Begriff Zielareal eingeführt werden. Von dem Begriff Zielareal sollen im Folgenden die mit dem zu desynchronisierenden Areal funktionell eng verbundene Neuronenpopulation und das verbindende Faserbündel verstanden werden.

Bei dem erfindungsgemäßen Stimulationsmechanismus wird innerhalb einer Periode der oszillatorischen Aktivität in der zu desynchronisierenden Neuronenpopulation über die einzelnen Elektroden 2 zu bestimmten, typischerweise unterschiedlichen Zeitpunkten stimuliert. Die Zeiträume zwischen diesen Einzelreizen werden als Bruchteile der Periode der oszillatorischen, zu desynchronisierenden Aktivität angegeben und betragen vorzugsweise im wesentlichen ein N-tel der Periode, wobei N eine kleine ganze Zahl, zum Beispiel 4, ist. N ist hierbei eine ganze Zahl, vorzugsweise unterhalb von 1000, besonders bevorzugt kleiner als 100 insbesondere kleiner als 10. Die Periode der zu desynchronisierenden oszillatorischen Aktivität, die als zeitliche Referenz für die Applikation der Einzelreize dient, wird im Folgenden als Stimulationsperiode T bezeichnet. Der Begriff der Stimulationsperiode T ist insofern zentral für das erfindungsgemäße Funktionieren, als mit dem in Abschnitt 5.1.2.2.2. beschriebenen Verfahren die Stimulationsperiode T weder durch Kalibrierung eingestellt, noch durch Messung während des Stimulationsbetriebs angepasst werden muss, sondern vielmehr der zu desynchronisierenden Neuronenpopulation aufgezwungen wird.

Unter einem Rhythmus wird die rhythmische, also nahezu periodische neuronale Aktivität verstanden, die sich in Folge einer krankhaft übersteigert synchronen Aktivität von Nervenzellen ergibt. Ein Rhythmus kann kurzzeitig auftretend oder lang anhaltend sein.

Unter einem Reset einer Neuronenpopulation wird der Reset, das heißt die Phasenrücksetzung, der oszillatorischen Aktivität dieser Neuronenpopulation verstanden.

3.3. Ausführungsform für den Fall, dass alle Elektroden in der zu desynchronisierenden Nervenzellpopulation positioniert sind:

Die N Elektroden sollen vorzugsweise so angeordnet sein, dass mit jeder einzelnen Elektrode ungefähr ein N-tel der zu desynchronisierenden Nervenzellpopulation stimuliert werden kann. Dies kann mit unterschiedlicher Anzahl der Elektroden und mit unterschiedlicher geometrischer Anordnung der Elektroden zueinander realisiert werden. Es kann beispielsweise eine beliebige, unsymmetrische Anordnung gewählt werden. Bevorzugt werden jedoch im wesentlichen symmetrische Anordnungen, da bei diesen die stimulationsbedingte funktionelle Aufteilung in Subpopulationen mit dem geringsten Stromeintrag ermöglicht wird. Beispielhaft können die Endpunkte der Elektroden, entlang der Elektroden projiziert, im wesentlichen ein Quadrat ergeben. Es können beispielsweise auch 6 Elektroden verwendet werden. Dabei liegen 4 vorzugsweise im wesentlichen quadratisch angeordnet in einer Ebene, während die anderen beiden im wesentlichen äquidistant senkrecht zu dieser Ebene liegen, wobei ihre Verbindungslinie im wesentlichen die Rotationsachse der 4 quadratisch angeordneten Elektroden bildet. Zur Verwirklichung verschiedener geometrischer Anordnungen können die Elektroden mindestens teilweise verschiedene Längen aufweisen.

3.4. Ausführungsform für den Fall, dass mindestens eine Elektrode 2 nicht in der zu desynchronisiernden Nervenzellpopulation positioniert ist:

Bei dieser Stimulationsform wird in mindestens einem, von dem zu desynchronisierenden Areal, verschiedenen Zielareal stimuliert. Hierbei kann, wie in Abschnitt 3.2 beschrieben, die indirekte Stimulation durch Stimulation einer von der zu desynchronisierenden Nervenzellpopulation verschiedenen Neuronenpopulation und/oder durch Stimulation eines mit der zu desynchronisierenden Nervenzellpopulation verbundenen Faserbündels erfolgen. Dabei kann in einem Zielareal, beziehungsweise in dem zu desynchronisierenden Areal, entweder mindestens eine Elektrode 2 oder eine in Abschnitt 3.3 beschriebene Mehrelektroden-Anordnung verwendet werden.

4. Bedarfsgesteuerte Applikation:

4.1. Muster und Polarität der Reize:

Im Rahmen der Applikation eines Reizes wird über jede einzelne Elektrode 2 ein Einzelreiz appliziert. Der Einzelreiz kann die in Abschnitt 2 beschriebenen Formen annehmen.

Die über die verschiedenen Elektroden 2 applizierten Einzelreize können, aber müssen nicht, bezüglich Art und/oder Energieeintrag verschieden sein. Zu diesem Zweck verfügt die erfindungsgemäße Vorrichtung über eine Steuerung, die so programmiert ist, dass sie die Art und/oder den Energieeintrag der Einzelreize variieren kann.

Die bei wiederholter Applikation eines Reizes über eine einzelne Elektrode 2 applizierten Einzelreize können, aber müssen nicht, bezüglich Art und/oder Energieeintrag variieren.

Beispielsweise können bei direkter Stimulation über N Elektroden 2 jeweils der gleiche Einzelreiz mit einer Zeitverzögerung von jeweils T/N appliziert werden, wobei T die Stimulationsperiode ist. Beispielsweise kann für N=4 in zeitlichen Abständen von jeweils T/4 der gleiche Einzelreiz nacheinander über die erste, zweite, dritte und vierte Elektrode 2 verabreicht werden, wie in Figur 5a-d dargestellt ist. Zu diesem Zweck verfügt die erfindungsgemäße Vorrichtung über eine Steuerung, welche so programmiert ist, dass sie N Elektroden 2 mit einer Zeitverzögerung von im wesentlichen *T/N* zur Einzelreizapplikation ansteuert.

Alternativ hierzu kann beispielsweise, insbesondere beim in Abschnitt 4.1 beschriebenen bedarfsgesteuerten Timing, die Reihenfolge der Einzelreize innerhalb eines Gesamtreizes systematisch oder zufallsgesteuert, das heißt, gemäß einer deterministischen oder stochastischen Regel, variiert werden. Hierzu verfügt die erfindungsgemäße Vorrichtung über eine Steuerung, die so programmiert ist, dass sie die Reihenfolge der Einzelreize innerhalb eines Gesamtreizes deterministisch und/oder stochastisch ansteuert.

Durch Variation der Reihenfolge der Einzelreize innerhalb der Gesamtreize kann Adaptationsvorgängen in den Neuronenpopulationen, die eine Erhöhung der Stimulationsintensität zum Erreichen der selben therapeutischen Wirkung bewirken, vorgebeugt werden.

Als weitere zusätzliche Möglichkeit können Zeitverzögerungen bei der Reizapplikation durch Wechsel der Polarität der Einzelreize ersetzt werden. Zu diesem Zweck verfügt die erfindungsgemäße Vorrichtung über eine Steuerung, die so programmiert ist, dass sie mindestens eine der Elektroden 2 mit jeweils wechselnder Polarität ansteuern kann. Zum Beispiel können für $N=4$ über die erste und zweite Elektrode 2 und nach einer Zeitverzögerung von T/4 über die dritte und vierte Elektrode 2 jeweils ein Paar von monophasischen oder biphasischen Einzelpulsen gegensätzlicher Polarität appliziert werden, wie in Figur 4 a-d für die monophasischen Einzelpulse gezeigt.

4.2. Nicht bedarfsgesteuerte Reizapplikation:

Die unter 4.1. beschriebenen Gesamtreize können in einer einfachsten Ausführungsform nicht bedarfsgesteuert appliziert werden. Dabei können diese Gesamtreize zeitlich streng periodisch oder zeitlich nicht periodisch verabreicht werden. In dieser Ausführungsform verfügt die erfindungsgemäße Vorrichtung über eine Steuerung, die so programmiert ist, dass sie eine nicht bedarfsgesteuerte Applikation der Gesamtreize ermöglicht. Die Steuerung ist dann so programmiert, dass sie dazu in der Lage ist, Gesamtreize periodisch und/oder nicht periodisch anzusteuern. Eine zeitlich nicht periodische Abfolge der Gesamtreize kann durch einen stochastischen oder deterministischen Prozess generiert werden.

4.3. Bedarfsgesteuertes Timing:

Unter Timing wird hier das zeitliche Muster der Reizapplikation bezeichnet.

Über Sensor 3 wird das Feedback-Signal gemessen, welches die Aktivität der zu desynchronisierenden Neuronenpopulation darstellt. Dieses Feedback-Signal wird an die Einheit 4 zur Signalverarbeitung und/oder Regelung weitergeleitet, die als Mittel zum Erkennen eines pathologischen Merkmals fungiert. Sobald die Einheit 4 zur Signalverarbeitung und/oder Regelung im Feedback-Signal ein pathologisches Merkmal erkennt, wird ein Reiz appliziert. Unter einem pathologischen Merkmal sind beispielsweise folgende Eigenschaften des Feedback-Signals zu verstehen:

a) Die Amplitude des Feedback-Signals überschreitet einen Schwellenwert. Die erfindungsgemäße Vorrichtung ist daher in einer bevorzugten Ausführungsform mit Mitteln zum Erkennen eines Schwellenwertes des Feedback-Signals ausgestattet. In diesem Fall wird vorzugsweise das Feedback-Signal selbst oder sein Betrag oder seine Amplitude mit dem Schwellenwert verglichen. Das Mittel zum Erkennen des Schwellenwertes kann bei dieser Ausführungsform so programmiert sein, dass es beispielsweise das Feedback-Signal selbst und/oder sein Betrag und/oder seine Amplitude mit dem Schwellenwert vergleicht. Die Bestimmung der Amplitude erfolgt entweder in einer einfachen Version mittels Bestimmung des Betrags des Signals, oder mit Bandpassfilterung und nachfolgender Hilbert-Transformation oder Wavelet-Analyse. Die Einheit 4 zur Signalverarbeitung und/oder Regelung ist in diesem Fall so programmiert, dass sie eine Bestimmung des Betrags des Signals und/oder eine Bandpassfilterung mit Hilberttransformation und/oder eine Wavelet-Analyse durchführen kann. Das Feedback-Signal oder sein Betrag wird besonders bevorzugt verwendet, da die Berechnung der Amplitude einen deutlich höheren Rechenaufwand bedeutet, und die Genauigkeit dieser Berechnung von der richtigen Auswahl algorithmischer Parameter abhängt. Außerdem kann die Bestimmung der Amplitude nicht auf einem einzelnen Messwert des Feedback-Signals durchgeführt werden, sondern muss in einem hinreichend großem, dem Fachmann bekannten Zeitintervall durchgeführt werden. Durch diese Form der Analyse des Feedback-Signals in einem gleitenden Zeitfenster ist die Erkennung des pathologischen Merkmals etwas verzögert. Die unter a) beschriebene Form der Analyse der Form des Feedback-Signals ist anzuwenden, wenn über Sensor 3 ausschließlich bzw. überwiegend die zu desynchronisierende pathologische Aktivität gemessen wird.

b) Falls über Sensor 3 neben dieser Aktivität zusätzlich noch nicht krankheitsspezifische Aktivität, zum Beispiel aus anderen Neuronenpopulationen, gemessen wird, muss bei der Analyse des Feedback-Signals ein weiterer algorithmischer Schritt eingefügt werden. Da die krankheitsspezifische Aktivität typischerweise in einem Frequenzbereich auftritt, der von dem Frequenzbereich der nicht krankheitsspezifischen Aktivität verschieden ist, genügt es hierzu vorzugsweise eine Abschätzung der Aktivität im krankheitsspezifischen Frequenzbereich durchzuführen. Die Frequenz der krankheitsspezifischen Aktivität wird beispielsweise durch eine Bestimmung der zeitlichen Differenz von aufeinanderfolgenden Triggerpunkten durchgeführt. Triggerpunkte sind charakteristische Punkte, wie Maxima, Minima, Wendepunkte und Nulldurchgänge. Vorzugsweise wird diese Analyse in einem gleitenden Zeitfenster durchgeführt, wobei der Mittelwert von mehreren zeitlichen Differenzen gebildet wird, wodurch die Stabilität der Frequenzschätzung erhöht wird. Alternativ kann die Frequenzschätzung auch mit den dem Fachmann bekannten spektralen Schätzmethoden und anderen Frequenzschätzern bestimmt werden.

Hierzu verfügt die erfindungsgemäße Vorrichtung in einer besonderen Ausführungsform Mittel zur Abschätzung der Aktivität im krankheitsspezifischen Frequenzbereich, wie spektrale Schätzmethoden, Wavelet-Analyse u.s.w. Dies wird beispielsweise durch eine Frequenzanalyse durch Mittel zum Durchführen einer Frequenzanalyse realisiert. Es kann beispielsweise die spektrale Energie im krankheitsspezifischen Frequenzbereich in einem gleitenden Fenster bestimmt werden. Alternativ kann nach Bandpassfilterung die Amplitude im krankheitsspezifischen Frequenzbereich durch Bestimmung des Maximums des bandpassgefilterten Signals oder durch Bestimmung des Mittelwerts des Betrags des bandpassgefilterten Signals oder mit nachfolgender Hilbert-Transformation oder mittels Wavelet-Analyse ermittelt werden. Hierzu umfasst die erfindungsgemäße Vorrichtung beispielsweise Mittel zur Bandpassfilterung der Amplitude und Mittel zur Bestimmung des Maximums des bandpassgefilterten Signals und/oder Mittel zur Bestimmung des Mittelwerts des Betrags des bandpassgefilterten Signals und/oder Mittel zur Durchführung einer Hilberttransformation und/oder einer Wavelet-Analyse.

Beim bedarfsgesteuerten Timing wird beispielsweise immer derselbe Reiz verwendet. Vorzugsweise wird die Stimulationsperiode T wie in Abschnitt 5.1.2.1. beschrieben an die momentane Frequenz der zu desynchronisierenden Neuronenpopulation angepasst. Es wird dann bei Vorliegen des pathologischen Merkmals ein Reiz mit an die momentane Frequenz angepasster Stimulationsperiode T appliziert. Die Intensität dieses Reizes bleibt dabei vorzugsweise konstant. Vorzugsweise wird die Intensität wie in Abschnitt 5.1.2.2.1. gemäß des Stimulationseffekts modifiziert.

4.4. Wiederkehrende Stimulation mit bedarfsgesteuerter Reizstärke:

Über Sensor 3 wird das Feedback-Signal gemessen, welches die Aktivität der zu desynchronisierenden Neuronenpopulation darstellt. Dieses Feedback-Signal wird an die Einheit 4 zur Signalverarbeitung und/oder Regelung weitergeleitet. Die Einheit 4 zur Signalverarbeitung und/oder Regelung führt eine wiederkehrende, vorzugsweise periodische Stimulation durch, wobei die Stärke der zum jeweiligen Zeitpunkt applizierten Reize von der Ausprägung des pathologischen Merkmals im Feedback-Signal abhängt. Zu diesem Zweck kann die Intensität oder die Dauer oder - bei Verwendung von Pulszügen - die Anzahl der Einzelpulse des Pulszugs an die Ausprägung des pathologischen Merkmals angepasst werden. In einem Zeitfenster frei wählbarer, vorzugsweise konstanter Länge, das in einem konstanten Zeitabstand vor dem jeweiligen Reiz endet, wird die Ausprägung des pathologischen Merkmals in folgender Weise ermittelt:

a) In dem Fall, wenn über Sensor 3 ausschließlich bzw. überwiegend die zu desynchronisierende pathologische Aktivität gemessen wird, entspricht die Amplitude der Ausprägung der Synchronisation im der zu desynchronisierenden Neuronenpopulation. Die Amplitude repräsentiert somit das pathologische Merkmal. Die Amplitude kann dabei abgeschätzt werden über die Bestimmung des Maximums des Signals oder über den Mittelwert des Betrags des Signals oder mit Bandpassfilterung mit nachfolgender Hilbert-Transformation oder Wavelet-Analyse. Die ersten beiden Varianten (Bestimmung des Maximums des Signals oder Bestimmung des Mittelwerts des Betrags des Signals) werden besonders bevorzugt verwendet, da die Berechnung der Amplitude mittels Hilbert-Transformation oder Wavalet-Analyse einen deutlich höheren Rechenaufwand bedeutet und deren Genauigkeit von der richtigen Auswahl algorithmischer Parameter abhängt.

b) Falls über Sensor 3 neben der krankheitsspezifischen Aktivität zusätzlich noch nicht krankheitsspezifische Aktivität, zum Beispiel aus anderen Neuronenpopulationen, gemessen wird, kann für die Abschätzung der Ausprägung des pathologischen Merkmals das Feedback-Signal nicht direkt angewandt werden. Da die krankheitsspezifische Aktivität typischerweise in einem Frequenzbereich auftritt, der von dem Frequenzbereich der nichtkrankheitsspezifischen Aktivität verschieden ist, wird in diesem Falle vorzugsweise eine Abschätzung der Aktivität im krankheitsspezifischen Frequenzbereich durchgeführt. Dies wird beispielsweise durch eine Frequenzanalyse realisiert. Es kann beispielsweise die spektrale Energie im krankheitsspezifischen Frequenzbereich bestimmt werden. Alternativ hierzu kann nach Bandpassfilterung die Amplitude durch die Bestimmung des Maximums des bandpassgefilterten Signals oder durch die Bestimmung des Mittelwerts des Betrags des Signals oder mit nachfolgender Hilbert-Transformation oder mit Wavelet-Analyse bestimmt werden.

4.5. Feststellung des Bedarfs:

Aus mindestens zwei Gründen gibt es keine eineindeutige Beziehung zwischen der Ausprägung des pathologischen Merkmals und der Ausprägung der krankheitsspezifischen Symptome. Zum einen bedingt die Entfernung von Sensor 3 zu dem Areal, in welchem das Feedback-Signal generiert wird, die Amplitude im krankheitsspezifischen Frequenzbereich. Zum anderen ist eine bestimmte Ausprägung des krankheitsspezifischen Merkmals, das heißt die Ausprägung der rhythmischen Aktivität im krankheitsspezifischen Frequenzbereich, nicht eineindeutig mit den krankheitsspezifischen Symptomen verbunden. Da der krankheitsspezifische Rhythmus Auswirkungen auf komplexe Nervennetzwerke im Gehirn hat, die typischerweise obendrein nicht einfachen linearen dynamischen Gesetzmäßigkeiten gehorchen, gelten keine eineindeutigen Relationen zwischen krankheitsspezifischem Rhythmus und Ausprägung der Symptome. Wenn zum Beispiel der krankheitsspezifische Rhythmus nicht hinreichend mit der biomechanisch vorgegebenen Eigenfrequenz einer Extremität übereinstimmt, ist der durch den krankheitsspezifischen Rhythmus bedingte Tremor deutlich geringer, als wenn der krankheitsspezifische Rhythmus in Resonanz mit der biomechanisch vorgegebenen Eigenfrequenz der Extremität übereinstimmt.

Die gemessene Aktivität befindet sich bei einer das Feedback-Signal erfassenden Lage des Sensors 3 in einem dem Fachmann bekannten Erfahrungsbereich. Der Wert der Ausprägung des krankheitsspezifischen Merkmals des über Sensor 3 gemessenen Feedback-Signals wird als Schwelle bezeichnet, bei dessen Überschreiten es typischerweise zum Auftreten von Symptomen, zum Beispiel des Tremors, kommt. Die Schwelle ist ein Parameter, der für die Ausführungsform des in Abschnitt 4.3. beschriebenen bedarfsgesteuerten Timings gewählt werden muss. Die erfindungsgemäße Vorrichtung umfasst daher Mittel zum erkennen eines Schwellenwertes. Mit dem erfindungsgemäßen Verfahren des bedarfsgesteuerten Timings wird der Vorteil erreicht, dass die Wirksamkeit der erfindungsgemäßen Vorrichtung nicht kritisch von der Wahl der Schwelle abhängt, sondern bezüglich der Wahl der Schwelle eine große Fehlertoleranz gegeben ist, die beispielsweise in einem Bereich von bis zu 50 % der maximalen Ausprägung des krankheitsspezifischen Merkmals liegt. Die Wahl der Schwelle wird entweder intraoperativ oder vorzugsweise in den ersten Tagen nach der Operation durch Messung des Feedback-Signals über Sensor 3, mit Bestimmung der Ausprägung des krankheitsspezifischen Merkmals und Vergleich mit der Ausprägung der Symptome, z. B. der Stärke des Zitterns.

In einer weniger bevorzugten Ausführungsform des bedarfsgesteuerten Timings wird als Schwelle ein repräsentativer Wert, zum Beispiel der Mittelwert, eines Kollektivs von bei Patienten gemessenen Schwellenwerten genommen.

In der in Abschnitt 4.4. beschriebenen Ausführungsform der wiederkehrenden Stimulation mit bedarfsgesteuerter Reizstärke ist keine Schwellendetektion notwendig.

5. Kalibrierung und Regelung:

5.1. Alle Elektroden 2 liegen in der zu desynchronisierenden Neuronenpopulation:

5.1.1. Stimulationsparameter zu Beginn der Stimulation:

5.1.1.1. Frequenz:

Wahl der Frequenz ohne vorherigen Betrieb der Vorrichtung:

Der Frequenzbereich der pathologischen neuronalen Aktivität ist für die jeweiligen Krankheitsbilder dem Fachmann bekannt (Elble R.J. und Koller W.C. (1990): Tremor John Hopkins University Press, Baltimore). Von diesem Frequenzbereich kann vorzugsweise der Mittelwert genommen werden. Alternativ kann statt dessen aus einer Datenbank der alters- und geschlechtsspezifisch zu erwartende Wert der Frequenz verwendet werden.

Es ist für den erfolgreichen Betrieb der erfindungsgemäßen Vorrichtung nicht notwendig, dass die anfänglich vorgegebene Frequenz mit der tatsächlich vorhandenen Frequenz der zu desynchronisierenden Neuronenpopulation übereinstimmt. Die unter 5.1.2.1. beschriebene Regelung der Stimulationsperiode T funktioniert auch, wenn ein vom richtigen Frequenzwert stark abweichender Anfangswert verwendet wird. Hierbei bedeutet stark abweichend, dass der Wert auch um mindestens einen Faktor 10 zu groß bzw. zu klein sein kann. Alternativ kann somit auch vorzugsweise mit einem Frequenzwert begonnen werden, der in dem Fachmann bekannten, für die Krankheit typischen Frequenzbereich liegt.

Wahl der Frequenz mit vorherigem Betrieb der Vorrichtung:

Als Startwert für die Frequenz wird der Mittelwert der Frequenz während des vorhergehenden Betriebs der Vorrichtung gewählt.

In beiden Fällen, das heißt mit und ohne vorherigen Betrieb der Vorrichtung, wird die Stimulationsperiode T berechnet als Kehrwert des Startwerts der Frequenz.

5.1.1.2. Intensität:

5.1.1.2.1. Bedarfsgesteuertes Timing:

Die Ausgangswerte der Stimulationsparameter, welche die Intensität der Einzelreize bestimmen (z. B. Länge des Hochfrequenzpulszugs, Amplitude und Dauer der Einzelpulse und Pause zwischen den Einzelpulsen) werden gemäß den dem Fachmann bekannten Erfahrungswerten (z. B. ein Hochfrequenzpulszug mit 10 Einzelpulsen, Dauer der Einzelpulse 60-200 $\mu$s, Rate der Einzelpulse 120 Hz, Amplitude 4 V) festgelegt.

Somit können die Anfangswerte für Frequenz und Intensität vorgegeben werden und müssen, insbesondere nicht im Rahmen einer zeitaufwendigen Kalibrierung, bestimmt werden.

5.1.1.2.2. Wiederkehrende Applikation mit bedarfsgesteuerter Reizstärke:

Die Ausgangswerte der Stimulationsparameter, welche die Intensität des Maximalreizes bestimmen (z. B. Länge des Hochfrequenzpulszugs, Amplitude und Dauer der Einzelpulse und Pause zwischen den Einzelpulsen) werden gemäß den dem Fachmann bekannten Erfahrungswerten (z. B. ein Hochfrequenzpulszug mit 10 Einzelpulsen, Dauer der Einzelpulse 60-200 μs, Rate der Einzelpulse 120 Hz, Amplitude 4 V) festgelegt.

Die Ausgangswerte der Stimulationsparameter, welche die Intensität des Minimalreizes bestimmen (z. B. Länge des Hochfrequenzpulszugs, Amplitude und Dauer der Einzelpulse und Pause zwischen den Einzelpulsen) werden gemäß den dem Fachmann bekannten Erfahrungswerten (z. B. ein Hochfrequenzpulszug mit 3 Einzelpulsen, Dauer der Einzelpulse 60-200 μs, Rate der Einzelpulse 120 Hz, Amplitude 4 V) festgelegt.

5.1.2. Regelmechanismen der erfindungsgemäßen Vorrichtung bzw. deren Steuerung während der Stimulation:

5.1.2.1. Anpassung der Stimulationsperiode T:

Im Zielareal oder einem damit eng verbundenen Areal wird das Feedbacksignal gemessen. Zum Beispiel kann beim Morbus Parkinson statt einer Messung über die Stimulationselektroden auch eine Messung der Aktivität in einem nachgeschalteten Areal, z. B. dem prämotorischen Cortex über epikortikale Elektroden erfolgen. In einem Zeitfenster mit unten angegebener Länge wird die dominante mittlere Frequenz bestimmt. Hierzu können unterschiedliche Algorithmen verwendet werden. Beispielsweise kann die Frequenz als Kehrwert der momentanen Periode bestimmt werden, wobei die momentane Periode durch die zeitliche Differenz zweier nachfolgender Maxima des Feedback-Signals gegeben ist. Falls über Sensor 3 nicht nur krankheitsspezifische Aktivität gemessen wird, muss für diese Art der Frequenzschätzung zuerst die krankheitsspezifische Aktivität über eine Bandpassfilterung des für die Krankheit spezifischen Frequenzbereichs extrahiert werden. Alternativ kann beispielsweise die Frequenz über die in Abschnitt 4.3 genannten Frequenzschätzer bestimmt werden. Die Stimulationsperiode T wird als Kehrwert der mittleren Frequenz festgelegt. Das für diese Frequenzschätzung verwendete Zeitfenster hat eine Länge, die nach oberen Werten offen sein kann und beispielsweise 10000 Perioden, vorzugsweise 1000 Perioden besonders bevorzugt 100 Perioden der krankhaften Aktivität aber auch anderen beliebigen Werten entspricht.

5.1.2.2. Bedarfssteuerung:

5.1.2.2.1. Bedarfsgesteuertes Timing:

Bei Überschreiten eines Schwellenwertes des Feedback-Signals erfolgt die jeweils nächste Stimulation mit vorzugsweise dem gleichen Reiz. Hierzu verfügt die erfindungsgemäße Vorrichtung über eine Steuerung, die nach Erkennen des Schwellenwertes mindestens ein stimulierendes Signal an die Elektroden 2 abgibt. Wird kein gewünschter Effekt erzielt, das heißt, wird die Zielpopulation nicht in ausreichendem Maße desynchronisiert und somit das Feedback-Signal nicht unter den Schwellenwert verschoben, wird die Stärke des Stimulus bis zu einem aus Sicherheitsgründen starr vorgegebenen Maximalwert, zum Beispiel 5V, langsam erhöht (z. B. in Schritten von 0,5 V pro 50 Perioden). Hierzu verfügt die erfindungsgemäße Vorrichtung über eine Steuerung, welche eine Änderung des Feedback-Signals erkennt und beim Ausbleiben der Änderung des Feedback-Signals die stimulierenden Signale nach oberen Werten anpasst. Nach ca. 20 erfolgreichen Stimuli kann die Vorrichtung beginnen, den Schwellenwert langsam (z. B. in Schritten von 0,5 V pro 200 Perioden) so lange nach oben regeln, solange der Stimulationserfolg noch vorhanden ist. Dabei wird der Stimulationserfolg wie in Abschnitt 4.5 beschrieben ermittelt. Die Steuerung ist dabei so programmiert, dass sie die Änderung des Feedback-Signals und somit den Stimulationserfolg erkennt.

5.1.2.2.2. Wiederkehrende Applikation mit bedarfsgesteuerter Reizstärke:

5.1.2.2.2.1. Schnelle Regelung:

Die Zeiträume zwischen den einzelnen Stimuli sind im wesentlichen ganzzahlige Vielfache der Stimulationsperiode T, das heißt, der Zeitraum zwischen dem Beginn oder bevorzugt dem Ende der Applikation zeitlich aufeinanderfolgender Stimuli ist gegeben durch:

$$t_{j+1} - t_j = N_j T \, .$$

Formel 1

Dabei ist $t_j$ der Zeitpunkt des Beginns oder bevorzugt des Endes des j-ten Reizes. T ist die Stimulationsperiode, und $N_j$ eine ganze Zahl. Der durch $t_{j+1}$-$t_j$ gegebene Zeitraum muss nicht, wie in Formel 1 definiert, streng einem ganzzahligen Vielfachen von T entsprechen, sondern kann davon auch gemäß

$$t_{j+1} - t_j = N_j T + x_j$$

Formel 2

gegeben sein, wobei $x_j$ möglichst klein ist im Vergleich zur Stimulationsperiode T. Die erfindungsgemäße Vorrichtung umfasst daher in einer Ausführungsform eine Steuerung, die vorzugsweise in Zeiträumen, die im wesentlichen ganzzahlige Vielfache der Stimulationsperiode T sind, Stimuli an die Elektroden 2 abgibt.
Hierbei sind prinzipiell alle denkbaren Variationen an zeitlichen Intervallen möglich, jedoch ist eine im wesentlichen streng periodische Applikation der Stimuli bevorzugt. Das heißt, bevorzugt ist die durch $N_1, N_2, N_3$ etc. gegebene Zahlenfolge eine konstante Zahlenfolge, das heißt, $N_j = N$ für alle $j=1,2,3$, etc. Die durch $N_1, N_2, N_3$, etc. gegebene Zahlenfolge kann aber auch von einer konstanten Zahlenfolge, abweichen. Beispielsweise kann die durch $N_1, N_2, N_3$, etc. gegebene Zahlenfolge periodisch, quasiperiodisch, chaotisch oder stochastisch sein.

Die Stärke des einzelnen Stimulus wird mit der erfindungsgemäßen Steuerung an die Ausprägung des pathologischen Merkmals des Feedback-Signals beispielsweise in folgender Weise angepasst:

In einem Zeitfenster vor der Applikation des Stimulus wird die Ausprägung des pathologischen Merkmals des Feedback-Signals wie in Abschnitt 4.4. abgeschätzt. Hierzu wird beispielsweise die Amplitude der oszillatorischen Aktivität im krankheitsspezifischen Frequenzbereich über eine Mittelung des Betrags des entsprechend bandpassgefilterten Feedback-Signals in einem Zeitfenster vor Stimulusapplikation bestimmt. Die Stärke des verwendeten Reizes wird bestimmt durch die Ausprägung des in Abschnitt 4.4. beschriebenen pathologischen Merkmals. Je stärker das pathologische Merkmal ausgeprägt ist, um so stärker ist der applizierte Reiz. Die erfindungsgemäße Steuerung ist daher in dieser Ausführungsform so programmiert, dass sie mit ansteigendem Feedback-Signal die Stärke des Reizsignals an die Elektroden 2, also den Energieeintrag erhöht. Die Beziehung zwischen der Ausprägung des Merkmals und der Reizstärke kann im einfachsten Fall linear sein, aber auch komplexer gestaltet werden, zum Beispiel nichtlinear sein.

Die Reizstärke kann durch Veränderung unterschiedlicher Stimulationsparameter, wie der Anzahl der Einzelpulse im Hochfrequenz- oder Niederfrequenz-Pulszug oder die Amplitude der Einzelpulse oder die Dauer der Einzelpulse, variiert werden. Bevorzugt wird die Anzahl der Einzelpulse im Hochfrequenzpulszug variiert.

Die Anzahl der Einzelpulse in dem Hochfrequenzpulszug, der über die k-te Elektrode 2 im Rahmen des j-ten Gesamtreizes appliziert wird, wird als $M_j^{(k)}$ bezeichnet.

Die Anpassung der Anzahl $M_j^{(k)}$ kann für die einzelnen Elektroden 2 separat durchgeführt werden. Bevorzugt wird aber die Anpassung für alle Elektroden 2 in gleicher Weise durchgeführt. Das heißt, es gilt $M_j^{(k)} = M_j^{(l)}$ für $k,l=1,2,3,...,N$, wobei N die Anzahl der Elektroden 2 ist. In diesem Fall wird die Anzahl der Einzelpulse des Hochfrequenzpulszugs mit $M_j = M_j^{(k)}$ für $k=1,2,3,...$, N bezeichnet. Die erfindungsgemäße Vorrichtung ist daher so programmiert, dass sie die Reizstärke in der angegebenen Weise variieren kann.

Wie in Abschnitt 4.4. beschrieben wird die Ausprägung des pathologischen Merkmals zum Beispiel über die Amplitude der oszillatorischen Aktivität im krankheitsspezifischen Frequenzband bestimmt. Hierzu wird zum Beispiel in einem Zeitfenster vor Applikation des j-ten Reizes der Betrag des im krankheitsspezifischen Frequenzbereich bandpassgefilterten Signals gemittelt. Die auf diese Weise bestimmte Größe wird als $A_j$ bezeichnet.

Der Zusammenhang zwischen der Anzahl der Einzelpulse im Hochfrequenzpulszug $M_j$ und der Amplitude $A_j$ kann zum Beispiel gegeben sein, durch

$$M_j = A_j \frac{M^{max}}{A^{max}} + M^{min}$$

$$\text{Formel 3}$$

wobei $M^{min}$ die Mindestanzahl der Einzelpulse im Hochfrequenzpulszug ist. Der Quotient $M^{max}/A^{max}$ ist neben $M^{min}$ der zweite einzustellende Parameter. $M^{max}$ als auch $A^{max}$ sind dem Fachmann bekannte Erfahrungswerte, woraus sich der Quotient $M^{max}/A^{max} = C$ ergibt. Durch Formel 3 wird die schnelle Regelung bestimmt, bei der für jeden Stimulus die Reizstärke, in diesem Falle die Anzahl der Einzelpulse des Hochfrequenzpulszugs $M_j$ an den aktuellen Wert der Amplitude $A_j$ angepasst werden.

5.1.2.2.2.2. Langsame Regelung:

Die oben angeführten Parameter $M^{min}$ und C können entweder manuell eingestellt werden oder von der erfindungsgemäßen Vorrichtung im Rahmen der langsamen Regelung eingestellt werden.

Die langsame Regelung kann auf einer Zeitskala stattfinden, die vorzugsweise zwischen 10 und 100 Perioden des Feedback-Signals entspricht. Dabei kann C als auch $M^{min}$ kombiniert sowie separat nach oben und unten variiert werden. Ziel dieser Regelung ist es, die Ausprägung des pathologischen Merkmals in dem Zeitfenster der langsamen Regelung ausreichend zu unterdrücken. Unter einer ausreichenden Unterdrückung des pathologischen Merkmals ist eine Unterdrückung unter die in Abschnitt 4.5. beschriebene Schwelle zu verstehen. Bevorzugt wird ausschließlich der Parameter $M^{min}$ geregelt.

5.2. Mindestens eine Elektrode 2 liegt nicht in der zu desynchronisierenden Neuronenpopulation:

Wie in Abschnitt 3.3 beschrieben, befindet sich mindestens eine Elektrode 2 nicht in der zu desynchronisierenden Neuronenpopulation. Im Falle einer nicht in der zu desynchronisierenden Neuronenpopulation gelegenen Elektrode 2, wird die zu desynchronisierende Neuronenpopulation über eine indirekte Stimulation beeinflusst, wie in Abschnitt 3.3 beschrieben. Da im Falle einer indirekten Stimulation die Leitungszeiten zwischen den stimulierten Neuronenpopulationen einerseits und der zu desynchronisierenden Neuronenpopulation andererseits jeweils verschieden groß sein können, werden vor der Durchführung der desynchronisierenden Stimulation zuerst die jeweiligen Leitungszeiten gemessen. Hierzu wird über jeweils eine Stimulationselektrode 2 gereizt und die Reizantwort über die, in der zu desynchronisierenden Neuronenpopulation plazierten Elektrode (Sensor 3), gemessen. Dies wird bei allen Stimulationselektroden 2, über die indirekt stimuliert wird, separat n mal durchgeführt, wobei n typischerweise eine kleine ganze Zahl bis zu beispielsweise 200 ist. Hieraus wird die mittlere Leitungszeit vorzugsweise in folgender Weise abgeschätzt:

Die Dauer zwischen Beginn der Stimulusapplikation über die j-te Elektrode 2 und dem ersten Maximum der Reizantwort bzw. des Betrags der Reizantwort, $\tau_j^{(k)}$, wird für jede einzelne Reizapplikation bestimmt. Bei $\tau_j^{(k)}$ steht der Index j für die j-te Elektrode 2, während der Index k für den k-ten applizierten Stimulus steht. Hieraus wird dann für jede Stimulationselektrode 2, über die indirekt stimuliert wird, separat die mittlere Dauer zwischen Reizbeginn und Reizantwort nach folgender Formel 4 bestimmt:

$$\overline{\tau}_j = \frac{1}{L_j} \sum_{k=1}^{L_j} \tau_j^{(k)} \; .$$

Formel 4

Hierbei ist $L_j$ die Anzahl der über die j-te Stimulationselektrode 2 applizierten Reize. $L_j$ kann, aber muss nicht für alle Stimulationselektroden 2, über die indirekt stimuliert wird, gleich sein.
Für die desynchronisierende Stimulation wird die auf diese Weise bestimmte Leitungszeit $\overline{\tau}_j$ in folgender Weise berücksichtigt:

Würde bei direkter Stimulation der zu desynchronisierenden Neuronenpopulation zur Zeit t über die j-te Stimulationselektrode 2 ein Reiz appliziert, so wird bei indirekter Stimulation über die j-te Stimulationselektrode 2 der Reiz zur Zeit $t - \overline{\tau}_j$ verabreicht.

Die Bestimmung der Stimulationsparameter zu Beginn der Stimulation und die Regelmechanismen während der Stimulation werden unter der oben beschriebenen Berücksichtigung der Leitungszeiten $\overline{\tau}_j$ völlig analog, wie in den Abschnitten 5.1.1. und 5.1.2. beschrieben, durchgeführt.

5.3. Bestimmung der Schwelle:

Der in Abschnitt 4.5. beschriebene Parameter der Schwelle muss für die Ausführungsform des in Abschnitt 4.3. beschriebenen bedarfsgesteuerten Timings gewählt werden. In einer bevorzugten Ausführungsform des bedarfsgesteuerten Timings wird die Schwelle entweder intraoperativ oder vorzugsweise in den ersten Tagen nach der Operation durch Messung des Feedback-Signals über Sensor 3, mit Bestimmung der Ausprägung des krankheitsspezifischen Merkmals und Vergleich mit der Ausprägung der Symptome z. B. der Stärke des Zitterns bestimmt. In einer bevorzugten Ausführungsform wird die Wahl der Schwelle in im wesentlichen regelmäßigen Abständen, zum Beispiel im Rahmen von halbjährlichen Kontrollen, überprüft.
In einer weniger bevorzugten Ausführungsform des bedarfsgesteuerten Timings wird als Schwelle ein repräsentativer Wert, zum Beispiel der Mittelwert, eines Kollektivs von bei Patienten gemessenen Schwellenwerten genommen.

5.4. Vorteile:

Die erfindungsgemäß durchgeführte Kalibrierung ist gegenüber der in der deutschen Patentanmeldung 102 11 766.7 beschriebenen Kalibrierung schneller, weniger störanfällig und weniger aufwendig. Sie ist deutlich schneller, da bei direkter Stimulation ohne Testreizung mit dem Stimulationsbetrieb begonnen werden kann, wobei im Laufe des Stimulationsbetriebs die Parameter wie in Abschnitt 5.1.2. optimiert werden. Bei wiederkehrender Stimulation mit bedarfsgesteuerter Reizstärke und direkter Stimulation der zu desynchronisierenden Neuronenpopulation ist keine Kalibrierung nötig. Im Gegensatz hierzu muss bei dem in der Anmeldung DE 102 11 766.7 beschriebenen Verfahren eine Serie von Testreizen, bei der Stimulationsparameter systematisch variiert werden, durchgeführt werden. Demgegenüber benötigt man für die Leitungszeitbestimmung für die oben beschriebene indirekte Stimulation typischerweise weniger als zwei Minuten. Erfindungsgemäß wird somit bei der beschriebenen Kalibrierung mindestens eine halbe Stunde Zeit gespart. Auf Grund der schnell durchführbaren Kalibrierung kann die erfindungsgemäße Methode schon intraopertiv angewandt werden, wodurch die Plazierung der Tiefenelektrode 2 optimiert wird. Es ist auf diese Weise möglich, die Auswirkung der desynchronisierenden Stimulation auf die Ausprägung der Symptome, zum Beispiel den Tremor, direkt als Parameter, für die Güte der Platzierung, zu verwenden.
Weniger störanfällig ist die erfindungsgemäße Kalibrierung im Vergleich zu der in der deutschen Patentanmeldung 102 11 766.7 beschriebenen Kalibrierung, da die im Rahmen der erfindungsgemäßem Kalibrierung verwendeten Frequenz- und Leitungszeitschätzer nicht kritisch von Parametern, wie zum Beispiel den Grenzen und der Charakteristik eines Bandpassfilters, abhängen.

Im Gegensatz hierzu hängt die Kalibrierung des in der deutschen Patentanmeldung 102 11 766.7 beschriebenen Verfahrens kritisch von den Parametern des verwendeten Bandpassfilters ab.

Außerdem sind die zur erfindungsgemäßen Kalibrierung verwendeten Frequenz- und Leitungszeitschätzer mit deutlich einfacheren Algorithmen realisierbar. Dementsprechend ist ihre software- bzw. hardwaremäßige Realisation deutlich weniger aufwendig.

Besonders vorteilhaft ist die Ausführungsform der wiederkehrenden Applikation mit bedarfsgesteuerter Reizstärke, da bei diesem Verfahren keine Schwelle detektiert werden muss. Im Gegensatz hierzu ist bei der Ausführungsform des bedarfsgesteuerten Timings als auch bei dem Verfahren der deutschen Patentanmeldung DE 102 11 766 eine Schwellendetektion notwendig.

Beispiel:

Wird z. B. an vier Orten stimuliert, so können über die vier Elektroden beispielhaft folgende Reize abgegeben werden:

1. Über jede der Elektroden wird derselbe Hochfrequenzpulszug appliziert, wobei, wie in Figur 5 a-d gezeigt die Pulszüge jeweils zeitlich um T/4 versetzt sind, wobei T die mittlere Periode des zu desynchronisierenden Rhythmus ist.

2. Über die Elektroden 1 und 2 werden, wie in Figur 4 a-d dargestellt, Hochfrequenzpulszüge gleicher Länge aber unterschiedlicher Popularität appliziert. Ebenso werden über die Elektroden 3 und 4 dieselben Hochfrequenzpulszüge appliziert, d. h. Für die Elektroden 1 und 3 bzw. 2 und 4 werden jeweils die gleichen Hochfrequenzpulszüge verwendet. Die Hochfrequenzpulszügen der Elektroden 3 und 4 werden um T/4 zeitlich versetzt (d. h. später) appliziert als die Hochfrequenzpulszüge der Elektroden 3 und 4.

Statt der Hochfrequenzpulszüge können jeweils auch Einzelpulse oder Niederfrequenzpulszüge (mit einer Frequenz im Bereich der Frequenz der zu desynchronisierenden Neuronenpopulation) verwendet werden.

Es gibt beispielhaft zwei unterschiedliche Kontrollmechanismen, mit denen eine bedarfsgesteuerte und somit energiesparende und milde (Nebenwirkungen vermeidende) Stimulation ermöglicht wird:

1. Bedarfsgesteuertes Timing (d. h. bedarfsgesteuerte Wahl der Zeitpunkte) der Applikation der Gesamtreize (Fig. 2): Immer, wenn die Synchronisation der Nervenzellpopulation einen Schwellenwert überschreitet, wird der nächste Gesamtreiz über alle Elektroden abgegeben. Diese Variante kann vorzugsweise dann herangezogen werden, wenn die Frequenz des zu unterdrückenden Rhythmus nicht zu stark schwankt.

2. Wiederkehrende Stimulation mit bedarfsgesteuerter Länge der Hochfrequenzpulszüge (Fig. 3):

Es erfolgt eine periodische Stimulation mit koordinierten Reizen über alle Elektroden. Dabei wird die Stärke der Reize, d. h. vorzugsweise die Länge der Hochfrequenzpulszüge, an die Stärke der Synchronisation der Neuronenpopulation angepasst: Je stärker die Synchronisation, desto stärker ist der koordinierte Reiz. Bei dieser Variante kann man als Zeitverzögerung zwischen den Einzelreizen (s.o.) statt T/4 vorzugsweise τ /4 wählen, wobei T die Periode des Rhythmus ohne Stimulation und τ die durch Stimulation dem Rhythmus aufgezwungene Periode ist. Mit anderen Worten: τ ist die Frequenz, mit der die Einzelreize appliziert werden. Hierdurch zwingt man dem System den einzigen kritischen Stimulationsparameter auf: Anstatt diesen im Rahmen einer aufwendigen Kalibrierung geeignet zu bestimmen, wird er durch die Stimulation diktiert. Außerdem wird bei dieser Form der bedarfsgesteuerten Stimulation der Umstand ausgenützt, dass die Neuronen in den betroffenen Gebieten eine (krankhafte) Tendenz zu periodischem Feuern bzw. Bursten (rhythmische Produktion von Gruppen von Aktionspotentialen) haben. Deswegen lässt sich leicht ein Entrainment erzielen, d. h. es ist einfach, dass man in Subpopulationen den periodischen Rhythmus stabilisiert. Aus diesem Grund benötigt diese Form der Stimulation im Vergleich zum bedarfsgesteuerten Timing ca. 1.5 mal weniger Strom.

Bei beiden Kontrollmethoden (bedarfsgesteuertes Timing und bedarfsgesteuerte Stärke) kann man vorzugsweise den einzigen wichtigen Stimulationsparameter, die Zeitverzögerung zwischen den Einzelreizen, durch Messung der Frequenz der Nervenzellpopulation im Zielgebiet oder einer anderen, damit eng verbundenen Nervenzellpopulation anpassen. Auch in diesem Fall hat die Methode 2 (bedarfsgesteuerte Stärke) den Vorteil, dass ihre desynchronisierende Wirkung stabiler ist gegenüber kleineren Fehlern bei der Frequenzschätzung oder abrupten Schwankungen der Frequenz.

Das Fehlen zeitaufwendiger Kalibrierung und die Stabilität der Wirkung auch bei stärkeren Frequenzschwankungen - insbesondere bei Methode 2 (bedarfsgesteuerte Stärke) - hat wichtige Konsequenzen:

1. Schon intraoperativ lässt sich beim Einführen der Tiefenelektrode der Stimulationserfolg sofort überprüfen. Hierdurch kann das Auffinden des geeigneten Zielpunkts deutlich verbessert werden. Für die bisherigen bedarfsgesteuerten Verfahren benötigt man eine Kalibrierung, welche pro Elektrode länger als 30 Minuten dauert. Das ist intraoperativ nicht durchführbar und dem (nicht narkotisierten) Patienten nicht zumutbar.

2. Die neuen Stimulationsmethoden lassen sich auch bei neurologischen bzw. psychiatrischen Erkrankungen anwenden, bei denen pathologische Rhythmen stark schwankende Frequenzen aufweisen. Insbesondere lassen sich mit den neuen Methoden auch intermittente (d. h. kurzzeitig auftretende) Rhythmen desynchronisieren. Hieraus ergibt sich, dass die neuen Stimulationsmethoden bei weit mehr Erkrankungen zur Anwendung kommen können, vor allem auch bei den Epilepsien.

Mit der erfindungsgemäßen Vorrichtung können mit dem neuen Stimulationsverfahren folgende Krankheiten bzw. Symptome durch Desynchronisation geeigneter Hirnareale behandelt werden.

Bei allen neurologischen und psychiatrischen Erkrankungen, bei denen pathologische neuronale Synchronisation eine für die Ausprägung der krankheitsspezifischen Symptome eine relevante Rolle spielt, zum Beispiel: Morbus Parkinson, essentieller Tremor, Dystonie, Zwangserkrankungen, Tremor bei Multipler Sklerose, Tremor in Folge eines Schlaganfalls oder einer anderen, zum Beispiel tumörösen Gewebsschädigung, zum Beispiel im Bereich des Thalamus und/oder der Basalganglien, Choreoathetose und Epilepsie, wobei die Aufzählung nicht einschränkend sein soll.

Bei der zur Zeit verwendeten Standardmethode, der Hochfrequenz-Dauerstimulation, werden folgende Zielareale beispielhaft verwendet:

Bei Morbus Parkinson der Nucleus subthalamicus oder bei tremordominantem Morbus Parkinson der Thalamus, zum Beispiel der Nucleus ventralis intermedius thalami.
Bei essentiellem Tremor der Thalamus, zum Beispiel der Nucleus ventralis intermedius thalami.
Bei Dystonie und Choreoathetose der Globus pallidum internum bei Epilepsie der Nucleus subthalamicus, das Kleinhirn, thalamische Kerngebiete, zum Beispiel der Nucleus ventralis intermedius thalami, oder der Nucleus caudatus.
Bei Zwangserkrankungen die Capsula interna oder der Nucleus accumbens.

Bei der erfindungsgemäßen Vorrichtung können beispielsweise die für die jeweiligen Erkrankungen oben aufgeführten Zielareale gewählt werden. Weil bei der erfindungsgemäßen Vorrichtung entweder keine Kalibrierung notwendig ist oder die Kalibrierung sehr schnell durchgeführt werden kann, ergibt sich die Möglichkeit, im Rahmen der Elektrodenimplantation alternative Zielareale auszutesten, bei denen sich die desynchronisierende Wirkung der erfindungsgemäßen Vorrichtung noch besser entfalten lässt.

Die Erfindung umfasst ebenfalls eine Steuerung, welche die angegebene Funktionsweise der erfindungsgemäßen Vorrichtung steuert sowie die Verwendung der Vorrichtung und der Steuerung für die Behandlung der Krankheiten Morbus Parkinson, essentieller Tremor, Dystonie, Zwangserkrankungen, Choreoathetose, Tremor bei Multipler Sklerose, Tremor in Folge eines Schlaganfalls oder einer anderen, zum Beispiel tumörösen Gewebeschädigung, zum Beispiel im Bereich des Thalamus und/oder der Basalganglien, und Epilepsie.

Die erfindungsgemäße Vorrichtung kann sowohl als Implantat zur dauerhaften Therapie der obengenannten neurologischen und psychiatrischen Erkrankungen als auch für die intraoperative Zielpunkt-Diagnostik, das heißt, die intraoperative Auffindung des optimalen Zielpunkts für die Elektrodenimplantation, verwendet werden.

**Patentansprüche**

1. Vorrichtung zur Desynchronisation der rhythmischen Aktivität von Hirnarealen umfassend Mittel zum Stimulieren von Hirnregionen, **dadurch gekennzeichnet, dass** sie folgende Komponenten umfasst:

   - N Elektroden (2) mit N $\geq$ 2, und
   - Steuerungsmittel, welche derart ausgestaltet sind, dass sie die N Elektroden (2) mit Steuersignalen so ansteuern, dass in den von den N Elektroden (2) stimulierten Subpopulationen einer zu desynchronisierenden Neuronenpopulation eine Phasenrücksetzung der neuronalen Aktivität bewirkt wird, wobei aufeinander folgende und von unterschiedlichen der N Elektroden (2) verabreichte phasenrücksetzende Reize um T/N zeitverschoben sind und T die Periode der rhythmischen, zu desynchronisierenden Aktivität ist.

**Claims**

1. An apparatus for desynchronising the rhythmic activity of brain areas including means for stimulating brain regions, **characterised in that** it includes the following components:

   - N electrodes (2), where N $\geq$ 2, and
   - control means which are configured such that they control the N electrodes (2) with control signals such that a phase reset of the neuronal activity is effected in the subpopulations of a neuronal population to be desynchronised, said subpopulations being stimulated by the N electrodes (2), wherein mutually following stimuli which are administered and phase reset by different ones of the N electrodes (2) are shifted in time by T/N, and wherein T is the period of rhythmic activity to be desynchronized.

**Revendications**

1. Dispositif pour la désynchronisation de l'activité rythmique de zones cérébrales, comprenant des moyens pour stimuler des régions cérébrales, **caractérisé en ce qu'**il comprend les composants suivants :

   - N électrodes (2) avec N ≥ 2, et
   - des moyens de commande qui sont configurés de telle sorte qu'ils pilotent les N électrodes (2) avec des signaux de commande tels que dans les sous-populations stimulées par les N électrodes (2) parmi une population de neurones à désynchroniser, ils provoquent une remise à zéro en phase de l'activité neuronale, dans lequel des excitations mutuellement successives stimulées par des électrodes différentes parmi les N électrodes (2) et assurant une remise à zéro en phase, sont décalées dans le temps à raison de T/N, et T est la période de l'activité rythmique à désynchroniser.

Figur 1

Fig. 2a

Fig. 2b

Fig. 3a

Fig. 3b

Fig. 4a

Fig. 4b

Fig. 4c

Fig. 4d

Fig. 5a

Fig. 5b

Fig. 5c

Fig. 5d

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10211766
- EP 1145736 A2
- US 5299569 A
- US 5540734 A
- US 2001051819 A1
- US 6529774 B1
- WO 9742990 A1

**In der Beschreibung aufgeführte Nicht-Patentliteratur**

- **P. TASS.** Desynchronizing double-pulse phase resetting and application to deep brain stimulation. *Biol. Cybern.,* 2001, vol. 85, 343-354
- **ELBLE R.J. ; KOLLER W.C.** Tremor. John Hopkins University Press, 1990